# EUROPÄISCHE PATENTSCHRIFT

(11) **EP 1 435 985 B1**
(45) Veröffentlichungstag und Bekanntmachung des Hinweises auf die Patenterteilung: **11.04.2007**
(21) Anmeldenummer: 01969710.1
(22) Anmeldetag: 12.09.2001
(51) Int. Cl.: A61K 38/00

(54) **ARZNEIMITTEL ENTHALTEND AKTIVIERTES ANTITHROMBIN III**
MEDICAMENT CONTAINING ACTIVATED ANTITHROMBIN III
MEDICAMENT CONTENANT DE L'ANTITHROMBINE III ACTIVEE

(30) Priorität: 12.09.2000 DE 10045047
(43) Veröffentlichungstag der Anmeldung: 14.07.2004
(73) Patentinhaber: Kehrel, Beate, 48149 Münster (DE)
(72) Erfinder: KEHREL, Beate, 48149 Münster (DE); BRODDE, Martin, 48153 Münster (DE)
(74) Vertreter: Böhm, Brigitte
(86) Internationale Anmeldenummer: PCT/EP2001/010541
(87) Internationale Veröffentlichungsnummer: WO 2002/022150

(56) Entgegenhaltungen:
- EP-A- 0 326 014
- EP-A- 1 027 894
- WO-A-02/058638
- O'REILLY M S ET AL: "ANTIANGIOGENIC ACTIVITY OF THE CLEAVED CONFORMATION OF THE SERPIN ANTITHROMBIN" SCIENCE, AAAS. LANCASTER, PA, US, Bd. 285, 17. September 1999 (1999-09-17), Seiten 1926-1928, XP002135982 ISSN: 0036-8075
- DICKNEITE GERHARD ET AL: "Reduction of mortality with antithrombin III in septicemic rats: A study of Klebsiella pneumoniae induced sepsis." THROMBOSIS AND HAEMOSTASIS, Bd. 69, Nr. 2, 1993, Seiten 98-102, XP009006685 ISSN: 0340-6245
- DATABASE WPI Section Ch, Week 199730 Derwent Publications Ltd., London, GB; Class B04, AN 1997-328454 XP002233002 & JP 09 132534 A (GREEN CROSS CORP), 20. Mai 1997 (1997-05-20)
- CAO YIHAI: "Endogenous angiogenesis inhibitors and their therapeutic implications." INTERNATIONAL JOURNAL OF BIOCHEMISTRY & CELL BIOLOGY, Bd. 33, Nr. 4, April 2001 (2001-04), Seiten 357-369, XP002233001 ISSN: 1357-2725

## Beschreibung

Die Erfindung betrifft die Verwendung von in seiner Konformation verändertem Antithrombin III, hier aktiviertes Antithrombin III (IDAAT= immune defence activated antithrombin) genannt, als Arzneimittel.

Antithrombin III ist ein wichtiger physiologischer Gerinnungsinhibitor, welcher, ohne dass es einer vorangehenden Aktivierung bedarf, zirkulierende Serinproteasen inhibiert.

Nach Komplexbildung spaltet die Protease die Arginin 393-Serin 394-Bindung, wodurch es zur Konformationsänderung von Antithrombin und zur Protease-Inhibitor-Komplexbildung kommt. Heparin beschleunigt durch Bindung im aminoterminalen Bereich von Antithrombin III die Antithrombin-Protease-Komplexbildung wesentlich. Es wird angenommen, dass in vivo Glykosaminoglykane, wie Heparansulfat an der Endotheloberfläche die Rolle des Heparins übernehmen.

Antithrombin III gehört zu der über 100 Mitglieder umfassenden Familie der Serinproteaseinhibitoren (Serpine) und ist ein Glykoprotein. Seine 432 Aminosäuren umfassende Polypeptidkette hat ein Molekulargewicht von 58000. Das Protein enthält drei intramolekulare Disulfidbrücken und vier Glykosilierungspositionen. In extrem hohen, unphysiologischen Dosen verabreicht, reduziert Antithrombin III im Tierexperiment die Mortalität der Sepsis (Dickneite and Paques, 1993). Bei an Sepsis erkrankten Menschen konnte aber keine signifikante Verbesserung der Mortalität oder Morbidität durch handelsübliche Antithrombin III-Präparate erreicht werden.

Neben der Serinproteasen-, insbesondere Thrombin-hemmenden Wirkung, wurde eine Erhöhung der Prostacyclin-Synthese in humanen und bovinen Endothelzellen durch Antithrombin III beobachtet (Yamauchi et al., 1989). Diese Erhöhung führte zu einer Suppression von Leukozyten (Kainoh et al., 1990) und wird durch Heparin beeinträchtigt (Uchiba et al., 1996), woraus geschlossen wurde, dass diese Wirkung des Antithrombin III über seine Bindung an Heparin-ähnliche Glycosaminoglykan-Rezeptoren vermittelt wird. Stangl et al 1999 beschrieben darüber hinaus eine leichte Steigerung (1.3-1.7-fache) der Freisetzung von Endothelin-1, bzw. Big-Endothelin 1 aus Lungengewebe von Ratten durch Antithrombin III.

Antithrombin III wird durch Entzündungs-mediierte Prozesse in seiner Form verändert. Die sogenannte "natürliche", "angeborene" oder "constitutive" Immunabwehr ist die erste Verteidigungsstrategie gegen "Eindringlinge" wie Bakterien, Viren, Parasiten etc. und ist im ganzen Tierreich verbreitet. Ein wichtiger Teil - dieser ersten Abwehr besteht darin, dass phagozytotische Zellen, insbesondere Monozyten und PMNL (neutrophile Granulozyten), aber auch Dendridische Zellen, Eosinophile, Blutplättchen und Mastzellen allein, oder als Assoziate mit anderen Zellen zum Ort des Eindringens des Pathogens wandern (Chemotaxis) und dabei durch Epithelien und Endothel penetrieren (Diapedese).

Am Ort der Entzündung werden die "Fremdzellen/Eindringlinge" durch Phagozytose neutralisiert. Die Entzündungszellen setzen dabei Proteasen, wie z.B. Elastase und Cathepsin G und Metalloproteasen und Substanzen frei, die eine Oxidation von Lipiden, Proteinen und Peptiden hervorrufen.

Zu diesen Substanzen gehören O₂, Superoxid, Hydrogenperoxid, Peroxynitrite, OH⁻-Radikale, Hypochlorige Säure HOCl, Cl₂-Gas, Chloramine. Die Halogenierung (hauptsächlich Chlorierung) ist dabei ein wichtiger Weg, Zellen abzutöten. Im Bereich der Entzündung wird der pH-Wert durch Freisetzung von Milchsäure auf bis unter pH 4.0 erniedrigt.

Die Abwehrzellen setzen auch spezifische Proteine und Peptide zur Abwehr frei, wie "bactericidal/permeability-increasing" (BPI) Protein aus Thrombozyten und Granulozyten und Defensine aus Granulozyten.

Besteht eine Wunde oder sonstige Aktivierung der Hämostase, so entstehen dabei Thrombin, Faktor Xa und andere Serinproteasen. Darüber hinaus kommt es zur Aktivierung von Complement (alternativer Weg, properdinpathway) und zur vermehrten Synthese und Freisetzung von sogenannten "Akute Phase Proteinen" wie Fibrinogen, C-reaktivem Protein, Mannose-Binding Protein (MBP), Produkten von sogenannten "immediate early genes" wie Thrombospondin-1, und anderen. Aktivierte Mastzellen setzen lösliches Heparin-Proteoglykan frei, welches an Antithrombin binden kann (Linstedt et al., 1992). Antithrombin III wird durch diese Vorgänge indirekt oder direkt verändert und erhält vollkommen neue Funktionen.

Im Rahmen der Erfindung wurde festgestellt, dass Antithrombin III, welches durch diese Vorgänge direkt oder indirekt verändert wird, vollkommen neue Funktionen erhält.

Im Rahmen der vorliegenden Erfindung wurde weiterhin festgestellt, dass Antithrombin III auch in vitro in diese aktivierte Form überführt werden kann, insbesondere durch Vorgänge wie Oxidation, Behandlung mit Harnstoff und Guanidinhydrochlorid, proteolytische Spaltung, Erwärmung auf 60°C, Erniedrigung des pH auf 4.0 oder Zusatz eines ATIII-Peptides, das die Sequenz SEAAAS enthält. Eine kryptische Sequenz des Antithrombin wird dabei freigelegt und erlaubt dem Protein die Interaktion mit Proteinen wie Thrombospondin, Vitronectin, CD36, oxLDL, αᵥβ₅-Integrin und anderen.

Im Rahmen der Erfindung wurde weiter festgestellt, dass aktiviertes Antithrombin lll (IDAAT) durch Selbstassoziation polymerisiert. Diese Polymere haben repetitive Bindungsstellen für die anhaftenden Proteine und immobilisieren diese. Anhaftende Proteine erhalten dadurch Funktionen, die sie als lösliche Proteine im Plasma, Serum oder anderen Körperflüssigkeiten nicht haben und Membranproteine können dadurch zur Signaltransduktion angeregt werden. Einer der wichtigsten Interaktionspartner für IDAAT ist Thrombospondin-1 (TSP-1). TSP-1 ist ein aus multiplen Domänen zusammengesetztes moduläres Glykoprotein, welches von vielen Zellen freigesetzt und in die extrazelluläre Matrix eingebaut wird. Insbesondere Blutplättchen enthalten hohe Konzentrationen an TSP-1 (Flicker und Kehrel, 1993) in ihren α-Granula und setzen es während ihrer Aktivierung frei.

Dabei steigt die lokale TSP-1-Konzentration um das mehr als 1000fache an (Flicker und Kehrel, 1993). Auch Endothelzellen, glatte Muskelzellen, Gliazellen und Leukozyten sezernieren TSP-1. TSP-1 ist Mitglied der Thrombospondin-Familie, der zusätzlich TSP-2, TSP-3, TSP-4 und das cartilage oligomeric matrix protein (COMP) angehören (Lawler et al., 1993). TSP-1 und TSP-2 sind in einigen Bereichen identisch, sodaß einige TSP-1 Funktionen auch von TSP-2 übernommen werden können. TSP-1 und TSP-2 haben die gleiche Domänen-Struktur und können als Homo- und Heteromere exprimiert werden (Bornstein et al., 1991). TSP-1 ist ein trimeres Glykoprotein mit einer aparenten Masse von 420000 Da. Seine 3 Untereinheiten weisen im Lämmli SDS-PAGE System (Lawler and Hynes 1986) eine molare Masse von 180000 Da auf. Elektronenmikroskopische Bilder zeigen die trimäre Struktur, die wie eine Bola aussieht mit globulären Enden an den Amino- und Carboxytermini der Polypeptidketten (Galvin et al. 1985). In der Nähe der globulären Aminotermini sind die drei Ketten durch Disulfidbrücken miteinander verbunden. Jede TSP-1-Untereinheit enthält 69 Cysteinreste, so dass jede Kette mindestens eine freie SH-Gruppe besitzt. TSP-1 und TSP-2 enthalten ähnliche funktionelle Domänen, wie die N-terminale Region, eine Pro-Kollagen homologe Region, Typ 1 TSP "repeats" (Wiederholungsbereiche), Typ 2 TSP "repeats", Typ 3 Calciumbindende "repeats" und die Carboxy-terminale Region (Bornstein et al., 1992).

Die stabförmigen Verbindungsregionen der TSP-1 Ketten zeigen eine Calcium-Abhängigkeit der Struktur. In Gegenwart von Ca²⁺ besitzt diese Struktur eine Länge von 16 bis 29.1 nm nach EDTA-Behandlung dagegen von 38.3 nm (Lawler 1986).

Die Konformation von TSP-1 ist stark abhängig von der Ca²⁺ -Konzentration (Lawler et al. 1988) und von der Bindung an Interaktionspartner. So verleiht die Bindung von TSP-1 an Fibronectin oder Heparin ihm eine Konformation in Abwesenheit von Ca²⁺, die das Molekül in Gegenwart von Ca²⁺ einnehmen würde (Dardik and Lahav 1999).

Immobilisiertes TSP, adsorbiert an Oberflächen, vermittelt die Adhäsion von Endothelzellen, glatten Muskelzellen und Monozyten. Diese Adhäsion ist abhängig vom Ca²⁺-Konformationszustand des TSP-1. EDTA-Behandlung inhibiert diesen Vorgang irreversibel (Lawler et al. 1988). Die Ca²⁺-Form des TSP-1 ermöglicht die Bindung an Zellen RGD-vermittelt über Integrine. Auch die Bindung an CD36 bewirkt eine Konformationsänderung im TSP-1-Molekül (Leung et al 1992). TSP-1 bindet an CD36 über einen zweistufigen Mechanismus. Erst im zweiten Schritt bindet TSP-1 mit hoher Affinität an CD36 über die "cell-binding site" im Properdin-ähnlichen "Type 1 repeat".

Bindung an CD36 über Peptidsequenz 139-155 des CD36 ermöglicht eine Konformationsänderung im TSP-1, welche die hochaffine Bindung an Sequenz 93-110 erlaubt. In diesem Bereich liegt die Sequenz von CD36, deren Bindungsfähigkeit durch Phosphorylierung/Dephosphorylierung (Thr 92) reguliert wird (Asch et al., 1993). Constitutiv phosphoryliertes CD36 bindet Kollagen, durch Zellaktivierung dephosphoryliertes CD36 erhält die Fähigkeit Thrombospondin zu binden. Die Konformation von TSP-1 reguliert seine Funktionsfähigkeit.

Neben der Fähigkeit an Zellen über Integrine zu binden und Zelladhäsion zu vermitteln, werden auch andere Fähigkeiten, wie z.B. die Fibrinolyse zu modulieren, Elastase und Cathepsin G zu inhibieren, die Wundheilung zu verbessern und das Auswachsen von Neuriten zu fördern, über die Konformation des TSP gesteuert.

TSP-1 defiziente Mäuse entwickeln zwischen der 1. und 4. Lebenswoche extensive akute und chronische organisierte bakterielle Lungenentzündungen mit massiver Einwanderung von Neutrophilen und Makrophagen. Diffuse alveolare Einblutungen wurden beobachtet. Zu einem späteren Infektionszeitpunkt kommt es im Vergleich zu Kontrollmäusen des gleichen Inzuchtstammes, die TSP-1 besitzen, zu einer Verdickung und Kräuselung des Epithels der Luftwege (Lawler et al. 1998).

Diese Ergebnisse verdeutlichen die Bedeutung des TSP-1 für die Abwehr von Infektionen. TSP-1 negative Mäuse produzierten signifikant weniger Nachwuchs als Kontrolltiere. TSP-1 "knock outs" zeigen eine ausgeprägte lordotische Curvatur der Wirbelsäule. Dies zeigt die Bedeutung des TSP-1 für die Entwicklung/Stabilisierung des Skeletts. TSP-1 defiziente Tiere hatten eine hochsignifikante höhere Anzahl von Leukozyten, insbesondere Monozyten und Eosinophile im periphären Blut.

TSP-1 ist ein multifunktionelles Protein. An Oberflächen immobilisiert fördert es die Bildung von Plasmin (Silverstein et al. 1986) und schützt durch Immobilisation gleichseitig das Plasmin vor Inaktivierung durch den alpha2 Plasmin-Inhibitor. Die hier beschriebene Erfindung, der Einsatz von IDAAT, bewirkt eine Immobilisierung von TSP-1 an Zelloberflächen. Der Urokinase Plasminogen Activator (uPA) und das "signal-chain" uPA (scuPA) binden an immobilisiertes TSP-1 und bleiben dabei proteolytisch aktiv. Die Bindung an immobilisiertes TSP schützt uPA vor der Inhibierung durch den Plasminogen Activator Inhibitor Typ 1 (PAI-1) (Silverstein et al., 1990).

Bindet scuPA an seinen Rezeptor (scuPAR) so wird eine Bindungsstelle frei, welche die Bindung an Zell-assoziiertes TSP-1 und Vitronectin (Vn) ermöglicht (Higazi et al., 1996). So ermöglicht immobilisiertes TSP-1 proteolytische Prozesse auch in einer Mikroumgebung, in der kein Fibrin vorhanden ist.

Zusammen mit Plasmin aktiviert immobilisiertes TSP-1 den "latent" transforming growth factor beta 1 (TGF-β-1) auf der Makrophagenoberfläche (Yehualaeshet et al., 1999).

Auch auf der Endotheloberfläche aktiviert TSP-1 das TGF-β (Schultz-Cherry and Murphy-Ullrich, 1993, Schultz-Cherry et al., 1994). TGF-β inhibiert die Proliferation von Endothelzellen und wirkt antiangiogenetisch. Inhibierung der Angiogenese durch TSP-1 wurde vielfach beschrieben (Iruela-Arispe et al., 1999, Jiminez et al., 2000). Auch eine Komplexbildung zwischen TSP und FGF-β1 (basic fibroblast growth factor) ist an dieser Funktion beteiligt (Murphy-Ullrich, 1993). Fehlen von TGF-β führt zu massiven Störungen der Infektabwehr, welche zum Tode führt (Kulkarni et al.,1993, Shull et al., 1992). Die TGF-β defizienten Tiere zeigten zusätzlich eine starke Autoimmunreaktivität (Letterio et al., 1996) über Beeinflussung von MHC class II Antigen-Expression (Geiser et al., 1993).

Da an Zelloberflächen immobilisiertes TSP-1 TGF-β aktivieren kann, liegt nahe, dass TSP-1 über TGF-β an den beschriebenen Prozessen der Infektabwehr und Autoimmunreaktivität beteiligt ist (Crawford et al., 1998). Immobilisiertes TSP-1 reguliert zusammen mit TGF-β die Proliferation von "Natural Killer"- Zellen (NK-Zellen) (Pierson et al., 1996). Die TSP-1 defizienten Tiere zeigen, wenn auch in schwächerer Ausprägung, entsprechende Immundefekte. Da das in dieser Erfindung erstmals beschriebene aktivierte Antithrombin, welches TSP-1 bindet, TSP an Zelloberflächen immobilisieren kann, liegt nahe, dass IDAAT indirekt die Aktivierung von TGF-β beeinflusst.

TSP-1 moduliert aber auch über andere Mechanismen immunologische Abwehr-relevante Prozesse. So haften eine groß Anzahl von Mikroorganismen, wie z.B. coagulase-negative Staphylococcen (Li et al., 2000), Enterococcen und Porphyromonas gingivalis fimbriae (Nakamura et al.,1999) an immobilisiertes TSP-1.

Mit dem Erreger der Malaria tropica befallene Erythrozyten haften an immobilisiertes TSP-1 (Roberts et al., 1985) und an den TSP-1 Rezeptor CD36.

Der Parasit selbst hat ein Membranprotein, welches TSP-1 homologe Bereiche enthält. Über dieses in die Erythrozytenmembran transportierte Protein TRAP (Thrombospondin-related-anonymus (adhesive)-protein) kann der Parasit das Anheften der befallenen Erythrozyten an die Gefäßwand vermitteln (Wegelnik et al., 1999, Kappe et al., 1999).

Auch andere Krankheitserreger, wie z.B. Cryptosporidium parvum oder Eimeria tenella, haben TSP oder TSP-Rezeptor homologe Domänen, die sie für die Zelladhäsion nutzen (Sulaiman et al., 1999).

Das HIV-1-Virus benutzt in seinem Oberflächenprotein GP 120 eine CD36 (TSP-Rezeptor)-Domäne, mit der der HIV Virus sowohl an TSP, als auch an CD4 auf den Wirtszellen binden kann (Crombie et al., 1998). Gereinigtes TSP-1 kann daher HIV-1 Infektionen inhibieren (Crombie et al., 1998).

Mehrere Complement-Proteine, C9, C8 alpha und C8 beta, haben Module mit hoher Homologie zu einem der "Repeat"-Module von Thrombospondin (Patthy, 1988). Weitere TSP-homologe Domänen haben auch Antistasin, Properdin und F-spondin. F-spondin ist, wie Thrombospondin selbst, eine effektive Substanz zur Verbesserung von Läsionen des Nervensystems (US 5750502).

Indem es gleichzeitig an apoptotische neutrophile Granulozyten (PMNL) und an Makrophagen bindet, kann TSP die Phagozytose von apoptotischen PMNL mediieren. Für diesen Prozess ist die gleichzeitige Interaktion von TSP mit seinen Rezeptoren αᵥβ₃ -Integrin, CD36 und CD47 verantwortlich (Savill et al., 1992). Die Phagozytose von apoptotischen PMNL reguliert Entzündungsreaktionen und verhindert ein unkontrolliertes Überschiessen. Im Gegensatz zur Phagozytose von nekrotischen PMNL oder zuweit im Abbau fortgeschrittenen PMNL erfolgt die TSP-1 vermittelte Phagozytose von apoptotischen PMNL ohne Freisetzung von proinflammatorischen Mediatoren (Stern et al., 1996).

Die rechtzeitige TSP-mediierte Phagozytose verhindert so eine überschießende Entzündungsreaktion. Zusätzlich wird aktiv von Makrophagen, welche apoptotische PMNL aufgenommen haben, die Produktion von proinflammatorischen Cytokinen inhibiert (Fadok et al., 1998). Die Quervernetzung vom TSP-Rezeptor CD47 auf Monozyten durch TSP-1, wie durch die hier beschriebene Erfindung erreicht, führt zusätzlich zu einer Inhibierung der Freisetzung von aktivem Interleukin 12 (IL-12) (Armant et al., 1999, Demeure et al., 2000). Interleukin-12 ist ein wichtiger Mediator der Sepsis (Steinhauser et al., 1999).

Die Immobilisierung von TSP-1 an apoptotische PMNL und an Monozyten ist daher ein guter Weg, medikamentös Zustände mit persistierenden, chronischen Entzündungen positiv zu beeinflussen. Zu diesen Erkrankungen gehören auch alle, bei denen eine überschießende Entzündungsreaktion einen Teil der Krankheit selbst darstellt, wie die verschiedenen Reperfusionsschäden, Abstoßungsreaktionen bei Organtransplantationen und Erkrankungen des rheumatischen Formenkreises.

Nicht nur apoptische neutrophile Granulozyten werden TSP vermittelt phagozytiert, sondern auch senescente Eosinophile (Stern et al., 1996). Dies zeigt, dass TSP-Immobilisierung an Zelloberfläche, wie durch die hier beschriebene Erfindung erreicht, auch ein Weg ist, die unerwünschte, proinflammatorische Antwort bei Erkrankungen, die Eosinophil-mediiert sind, wie Allergie, Asthma, parasitäre Erkrankungen, bestimmte Tumore und Bindegewebserkrankungen, medikamentös zu bekämpfen. So wird verhindert, dass hoch toxische Substanzen von den Eosinophilen freigesetzt werden und das Gewebe schädigen, bzw. zerstören.

TSP bindet Chemokine, wie RANTES, und verhindert so die Bindung des Chemokins an seinen Rezeptor (Barnes et al., 1998). Auch über diesen Weg moduliert TSP Entzündungsreaktionen und Immunabwehr.

Ein Medikament, welches TSP in seiner Funktion beeinflusst, indem es die Konformation ändert, oder die Immobilisation an Zelloberflächen von immunkompetenten Zellen fördert, limitiert die unerwünschte Immunresponse bei Krankheiten, wie, aber nicht beschränkt auf, Rheumatische Arthritis, Goodpasture Syndrom, Insulin-abhängiger Diabetes, Pemphigus, Pemphigoid, primäre biliäre Cirrhose, Colitis ulcera, Lupus erythematosus, Graft-versus-Host Erkrankung, Sepsis.

Immobilisierung von TSP an Zelloberflächen führt zur Quervernetzung seines Rezeptors CD47. Diese Quervernetzung von CD47 auf chronisch lymphatischen Leukämiezellen (CLL-Zellen) verursacht spezifisch den Zelltod dieser Tumorzellen (Mateo et al., 1999).

Eine Substanz, die die Bindung von TSP an Leukämiezellen bewirkt, wäre somit ein wirkungsvolles Medikament zur Bekämpfung der CLL, einer tödlichen Erkrankung, gegen die es noch kein spezifisches wirksames Medikament gibt.

Thrombospondin inhibiert nicht nur über seine Wirkung auf TGF-β die Neoangiogenese, sondern auch über Immobilisierung und Bindung an und Aktivierung von CD36.

Behandlung von Tumoren in Mäusen mit TSP-1 führt zu Inhibition der Neoangiogenese und zur Apoptose von Endothelzellen (Jiminez et al., 2000).

Inhibierung der Tumorangiogenese ist ein guter Weg, medikamentös das Wachstum von Tumoren zu begrenzen (Roberts et al., 1996). Eine Substanz, die die Bindung von TSP an Endothelzellen in Tumoren vermittelt, gewinnt damit antiangiogenetische und somit Krebsbekämpfende Eigenschaften.

Neoangiogenese ist auch eine Ursache für Erblindung, z.B. bedingt durch Diabetes mellitus (Kaplan et al., 1999, Shafiee et al., 2000), altersbedingte Macular Degeneration oder Frühreife von Säuglingen. Auch zur Bekämpfung dieser Neoangiogenese wäre eine Substanz, welche die Bindung von TSP an Endothelzellen vermittelt, medikamentös einsetzbar.

Nach einer Verletzung steigen die Konzentrationen an TSP im Gewebe rund um die verletzte Region signifikant an. Nach einer Balloon-Kathetisierung z.B. kann schon 1 Stunde nach Verletzung auf den Oberflächen der Zellen TSP detektiert werden (Watkins et al., 1990, Munjal et al., 1990). Das TSP auf der Zelloberfläche nimmt noch in den darauffolgenden Tagen zu und wird dann zunehmend auch in der Matrix angereichert.

Mit zunehmender Wundheilung verschwindet TSP wieder von den Zellmembranen des verletzten Gewebes.

Eine der Aufgaben, die TSP in der Wunde wahrnimmt, ist die Verbesserung der Wundheilung (US Patent 5, 155, 038). Eine Substanz, die TSP-1 in der Wunde immobilisiert, müsste die Wundheilung verbessern.

TSP in der Wunde wird von den Gewebezellen exprimiert aber auch von Blutplättchen, während deren Aktivierung freigesetzt.

Etwa 1 % des gesamten Plättchenproteins und ca. ¼ des Proteingehalts der Plättchen α-Granula ist Thrombospondin-1 (Kehrel, et al., 1996). Freigesetztes Thrombospondin fördert die Kollagen-induzierte Plättchenaggregation (Kehrel, et al., 1988). Im C-Terminus enthält TSP eine Sequenz, RFYVVMWK, welche über CD47 Plättchen aktiviert (Chung et al., 1999 und 1997). Lösliches TSP, dem Blut, Plättchensuspension oder Plättchenreiches-Plasma zugesetzt wird, löst jedoch allein keine Aggregation aus.

Während Plättchen in Suspension TSP nur in seiner Ca²⁺-Form binden können, haften Plättchen sowohl an die "high" als auch "low"-Ca²⁺-Form des an Matrix immobilisierten Thrombospondin.

Die Aufgabe der vorliegenden Erfindung war es daher, ein Arzneimittel bereitzustellen, welches die zuvor angesprochenen Funktionen ausüben und damit die erwarteten Wirkungen erfüllen kann.

Gelöst wird diese Aufgabe durch ein Arzneimittel, enthaltend aktiviertes Antithrombin III (IDAAT), IDAA T-Peptide.

Im Rahmen der vorliegenden Erfindung ist, wie oben bereits ausgeführt, festgestellt worden, dass aktiviertes IDAAT über seine nun neu im Rahmen der vorliegenden Erfindung festgestellten Eigenschaften und Funktionen eine Vielzahl von Reaktionen im Körper auslöst oder vermittelt, welche sich zur Behandlung von Krankheiten ausnutzen lassen. Diese Funktionen und Eigenschaften werden im Folgenden weiter erläutert werden, ebenso wie die damit zu behandelnden Krankheiten oder Krankheitszustände. Auch eine Prophylaxe kann in vielen Fällen mit Hilfe von IDAAT stattfinden.

Im Rahmen der vorliegenden Erfindung kann das Arzneimittel sowohl das vollständige IDAAT enthalten, welches beispielsweise gemäß dem in den Beispielen beschriebenen Verfahren hergestellt werden kann. Theoretisch kann auch aus dem Körper nach Abwehrreaktion gebildetes IDAAT isoliert werden. Des Weiteren ist es denkbar, IDAAT-Peptide zu verwenden, welche die Interaktion mit Proteinen wie Thrombospondin, Vitronectin, CD36, oxLDL, α_{IIb}β3-Integrin, αᵥβ3-Integrin und anderen vermitteln. Derartige geeignete Peptide können leicht durch vorbereitende Versuche aufgefunden werden, indem beispielsweise ihre Interaktion mit einem der oben genannten Proteine getestet wird.

Auch Analoga von IDAAT die ebenfalls die Interaktion mit den genannten Proteinen vermitteln sind im Rahmen der vorliegenden Erfindung beschrieben, IDAAT-Mimetika sind auch beschrieben welche aufgrund ihrer Struktur oder/und funktioneller Gruppen gleiche Wirkungen und Interaktionen wie IDAAT zeigen können.

Im Rahmen der vorliegenden Erfindung ist es bevorzugt, rekombinantes IDAAT zu verwenden, wozu rekombinant hergestelltes Antithrombin III in geeigneter Weise behandelt wird, um darauf aktiviertes Antithrombin III zu erhalten (siehe Beispiele sowie diese Beschreibung). Auch Peptide oder Analoga von IDAAT werden vorzugsweise in rekombinanter Form synthetisiert und dann aktiviert.

Ein erfindungsgemäßes Arzneimittel kann selbstverständlich weitere pharmazeutisch annehmbare Hilfs- oder/und Trägersubstanzen enthalten, wobei das Arzneimittel zur lokalen, intradermalen, oberflächlichen, intraperitonalen, intravenösen oder intramuskulären oder oralen Verabreichung formuliert wird oder seine Verabreichung über Vesikel ermöglicht wird. Das erfindungsgemäße Arzneimittel enthält daher vorzugsweise solche Hilfs- und Trägersubstanzen, die die jeweilige bevorzugte Applikationsart ermöglicht.

Das erfindungsgemäße Arzneimittel kann neben dem IDAAT davon weitere Substanzen enthalten, wie beispielsweise Antibiotika, Immunsuppressiva etc. Je nach zu behandelnder Krankheit kann es von Vorteil sein, unterstützend mit bekannten Arzneimitteln zu behandeln. Eine entsprechende Kombination dieses Arzneimittels mit IDAAT oder seinen Analoga ist daher gegebenenfalls eine bevorzugte Ausführungsform der vorliegenden Erfindung.

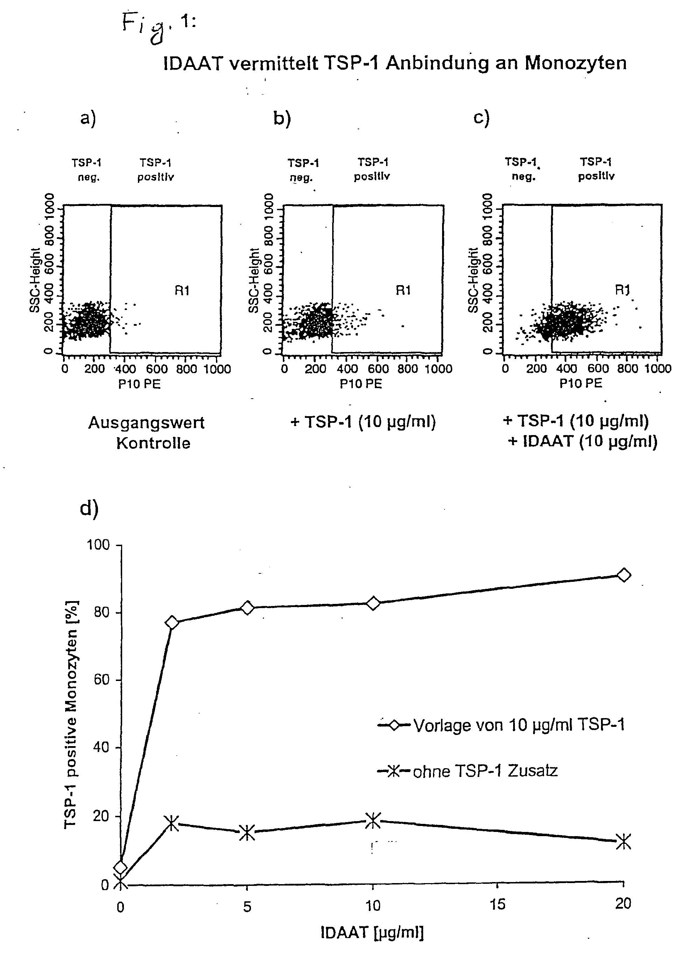

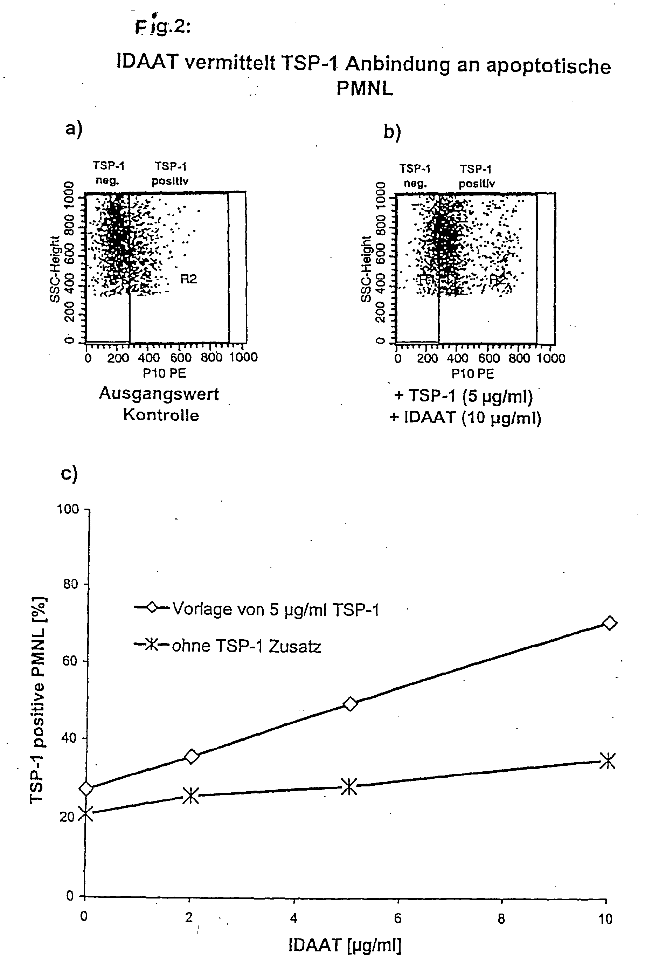

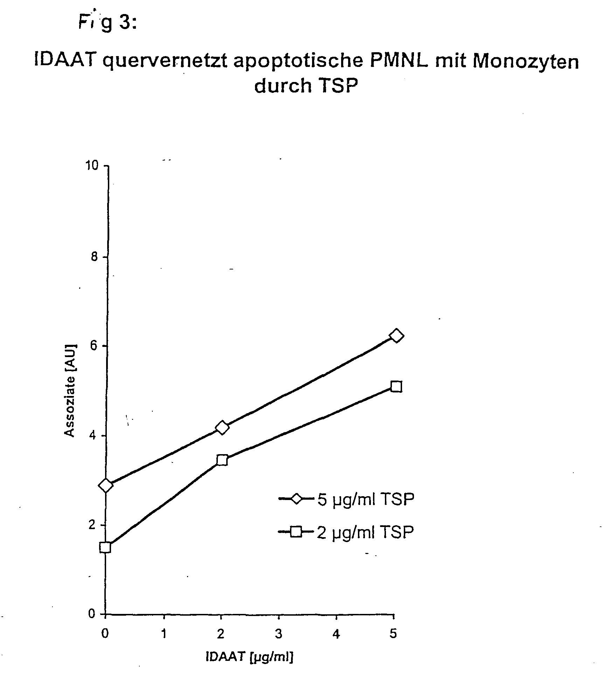

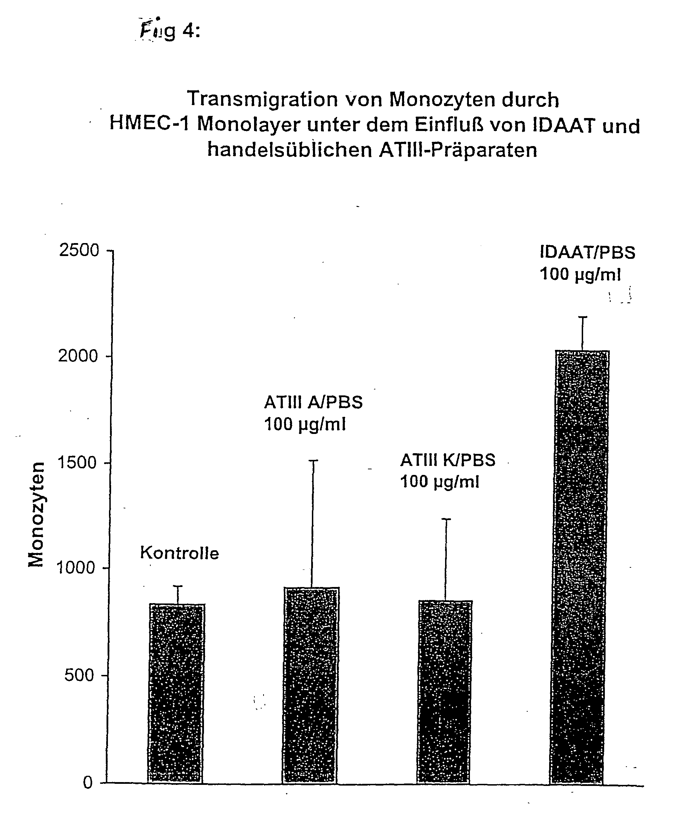

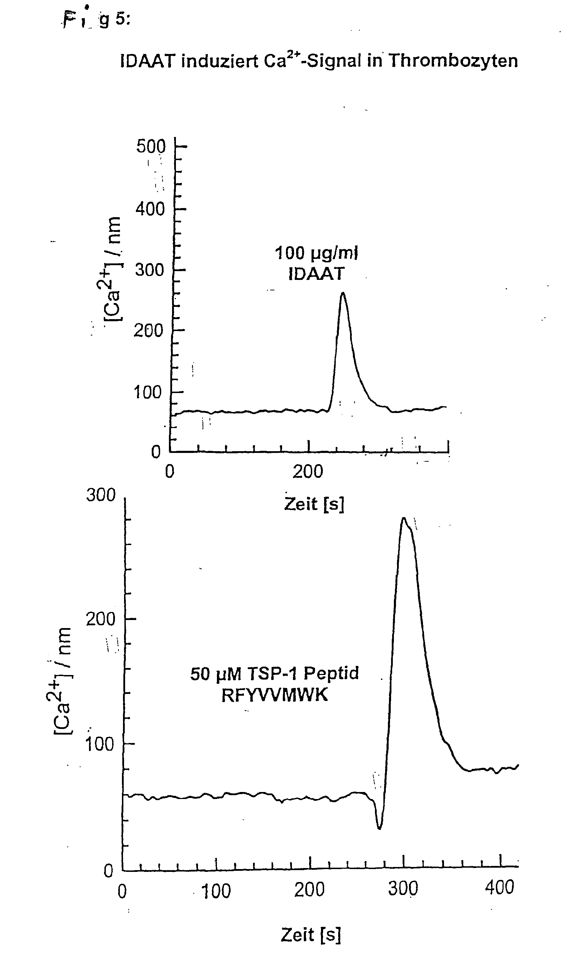

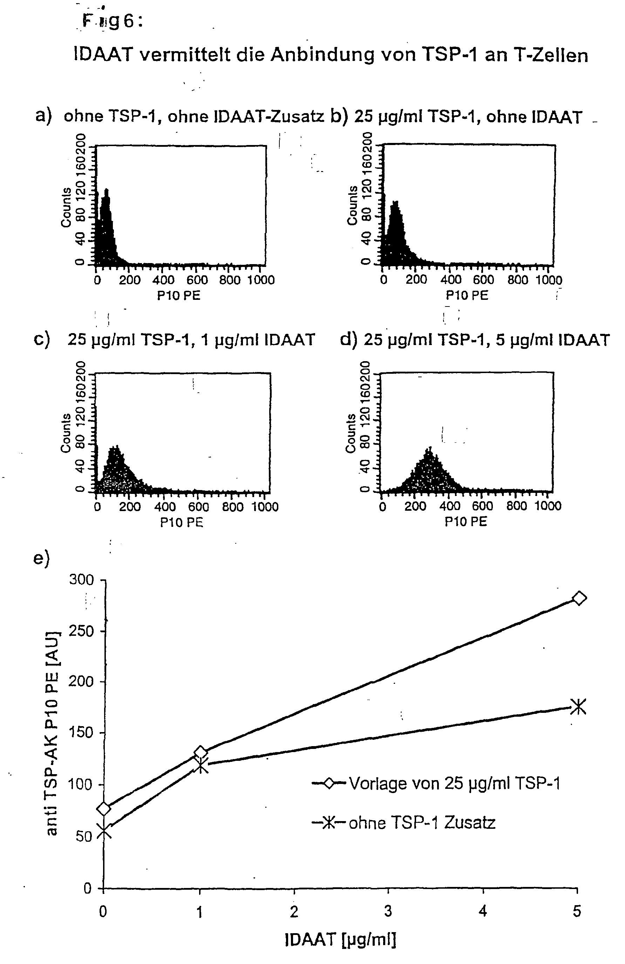

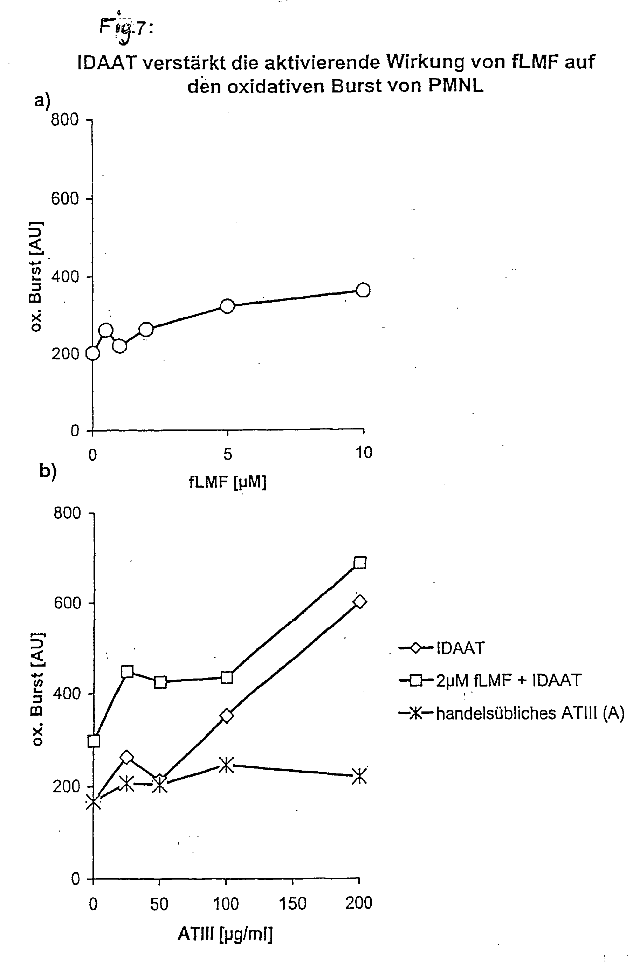

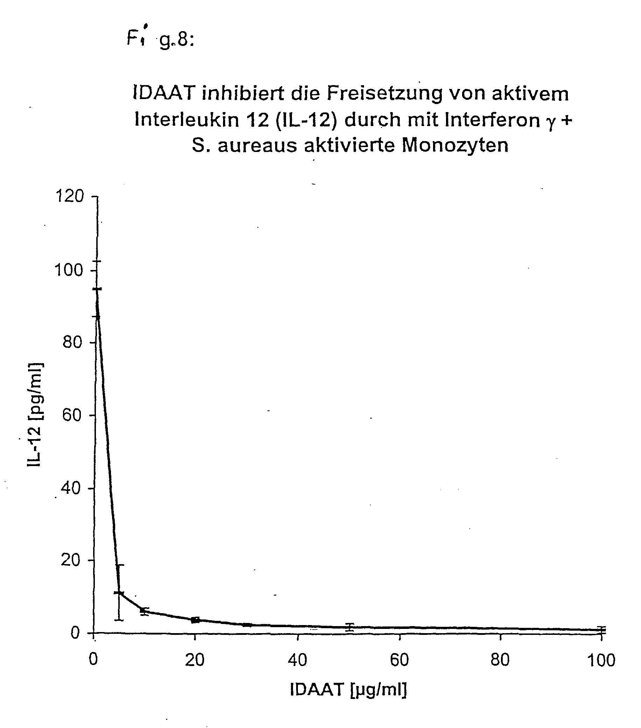

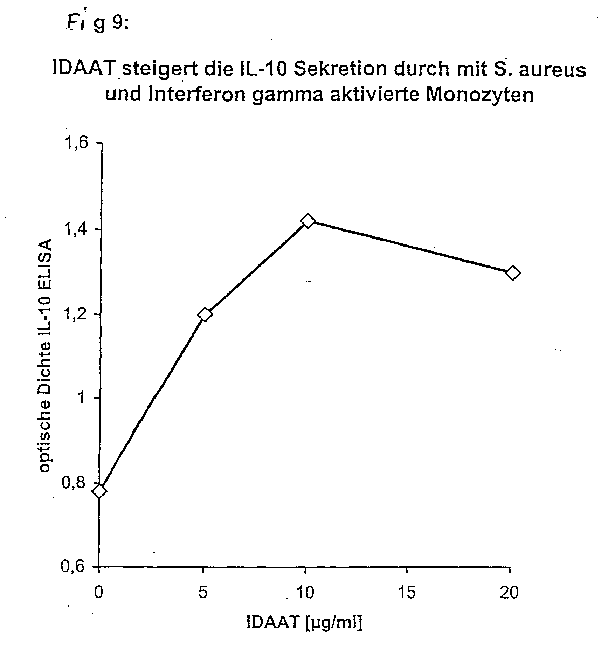

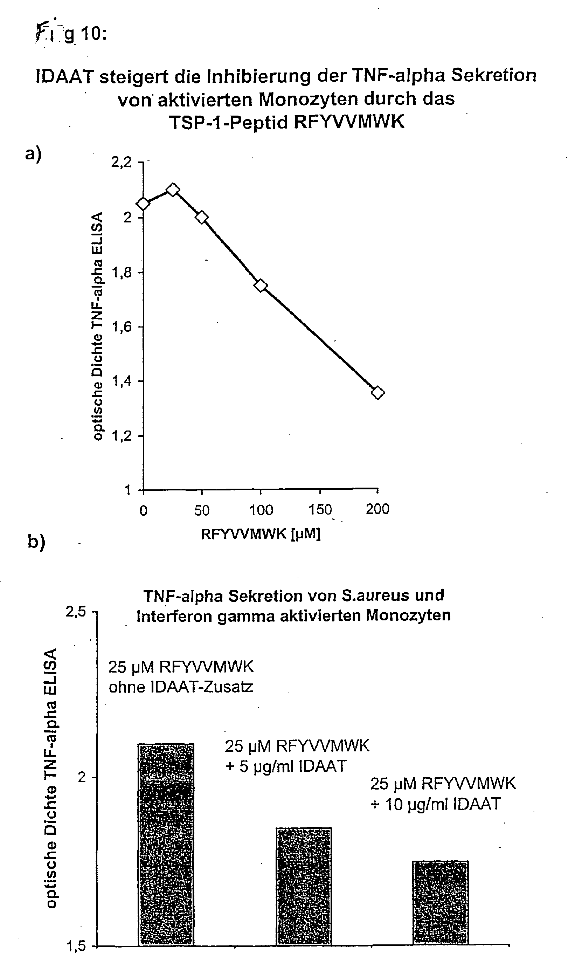

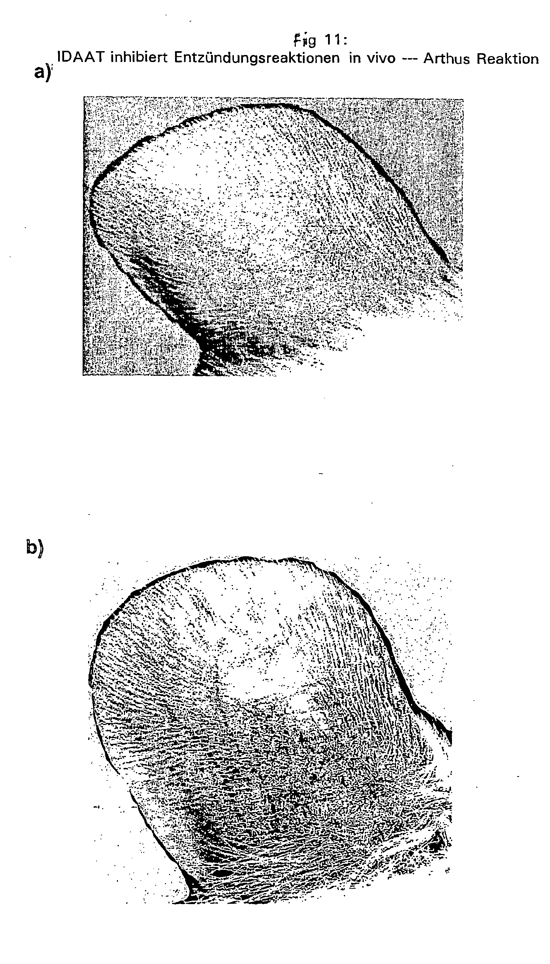

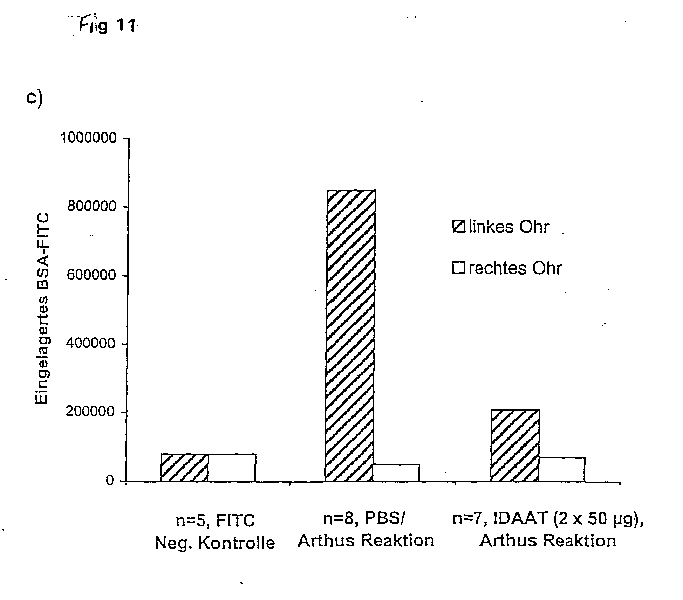

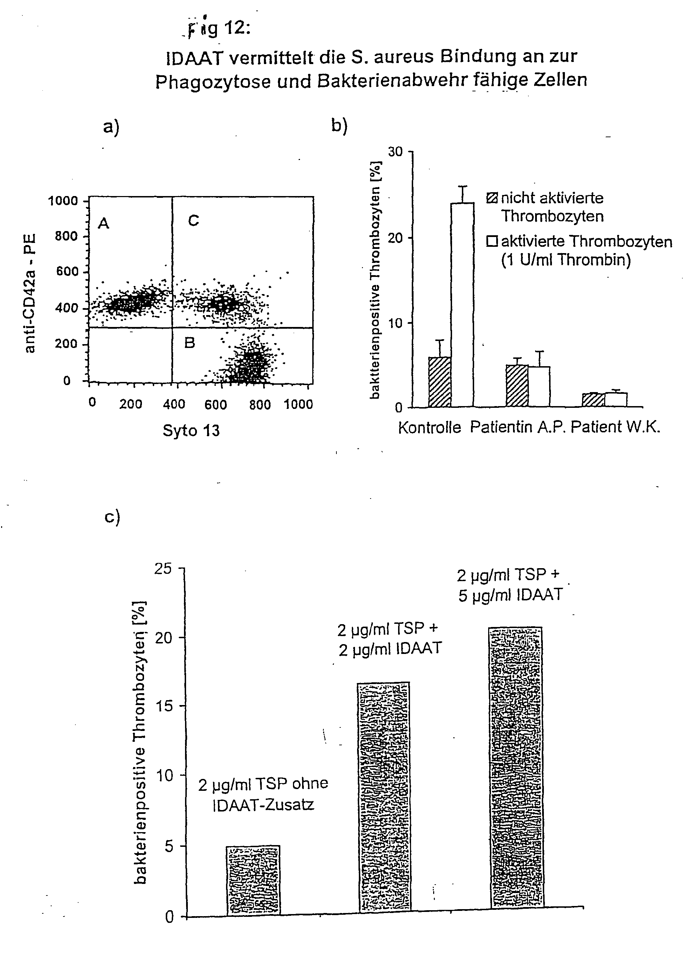

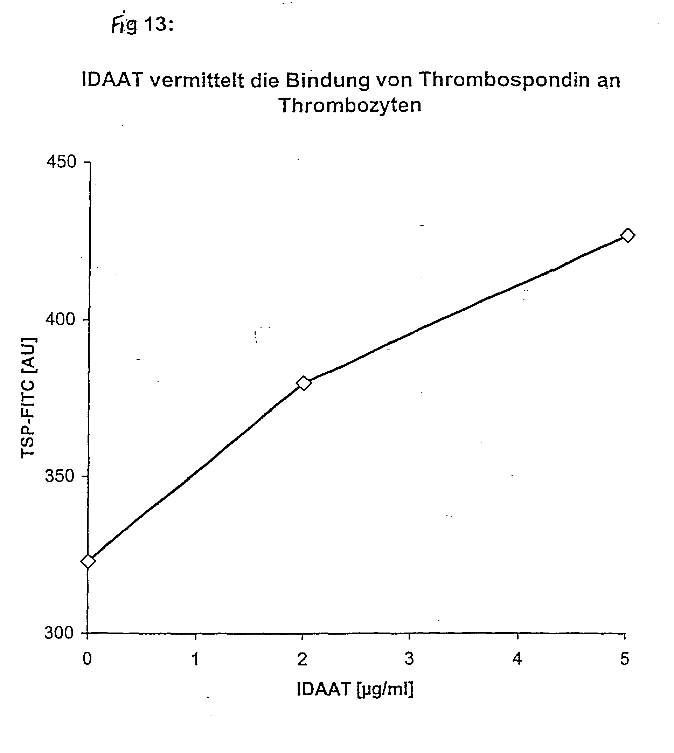

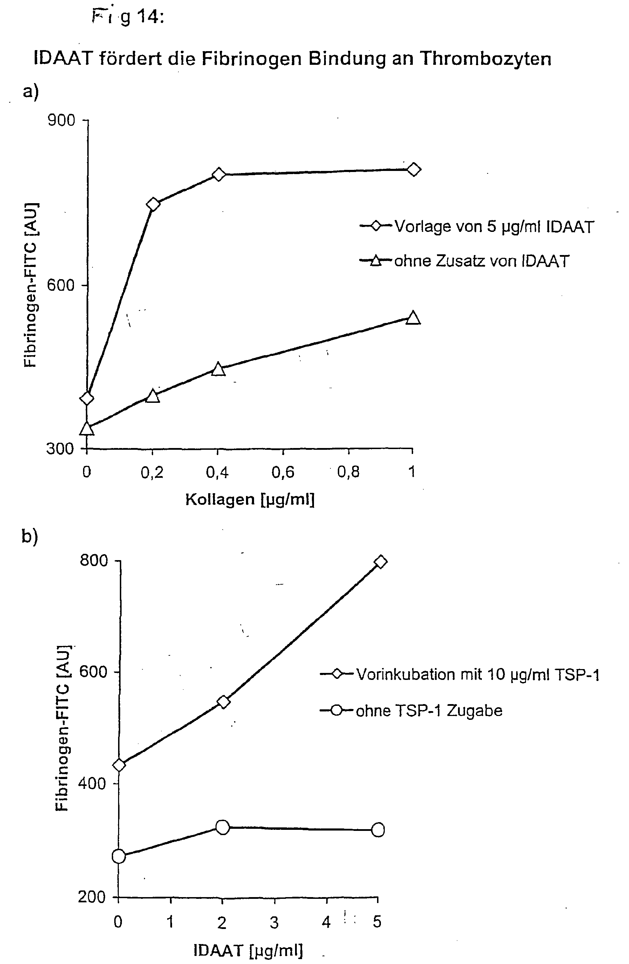

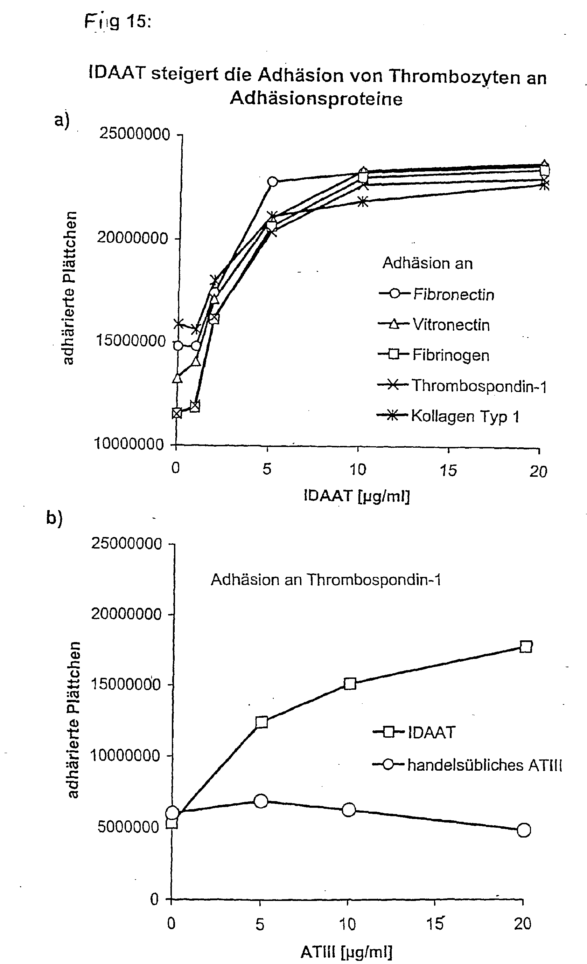

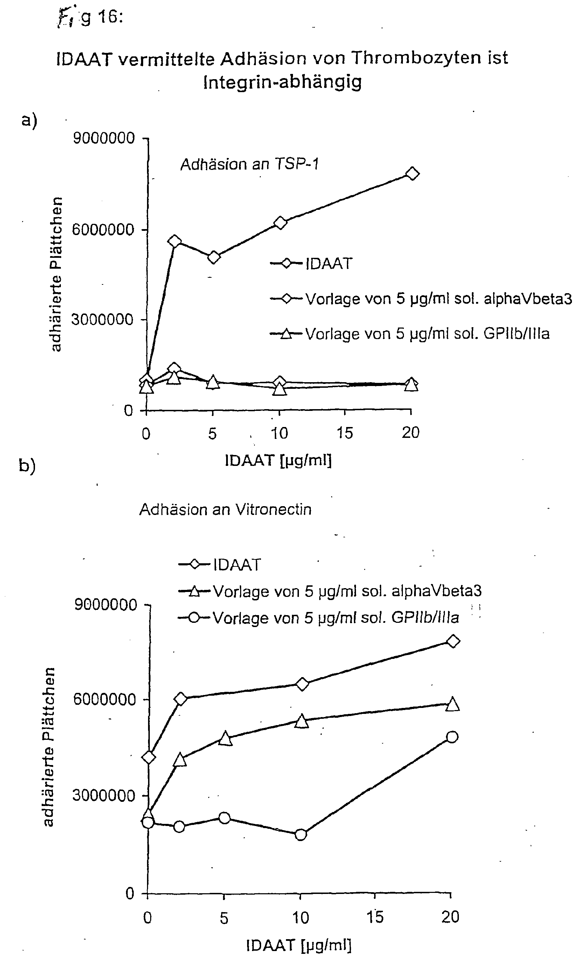

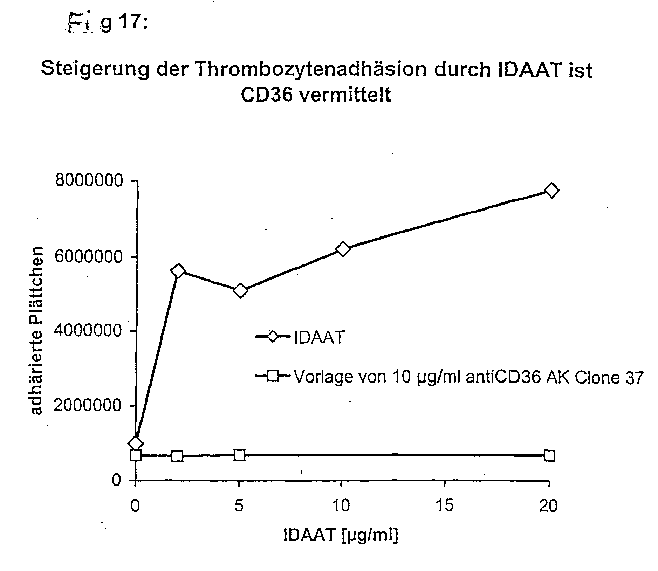

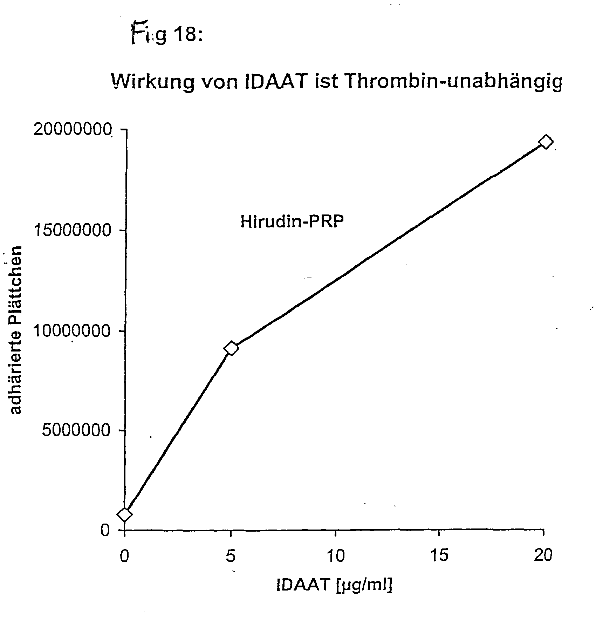

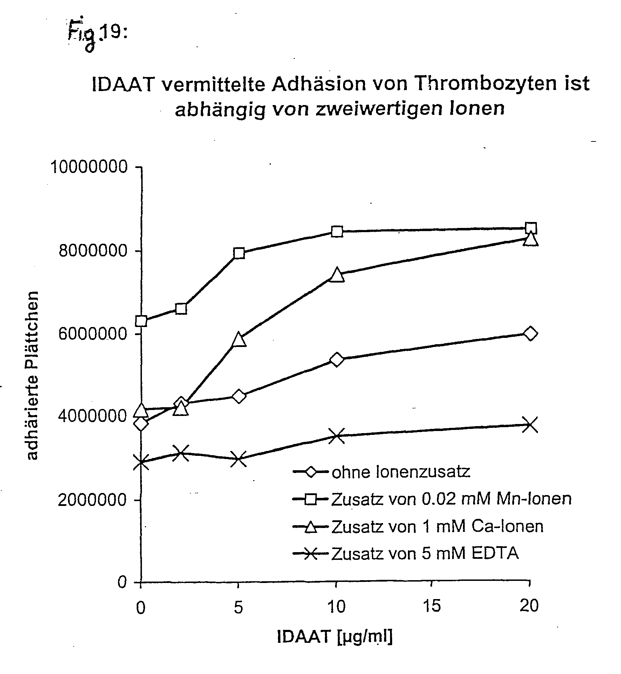

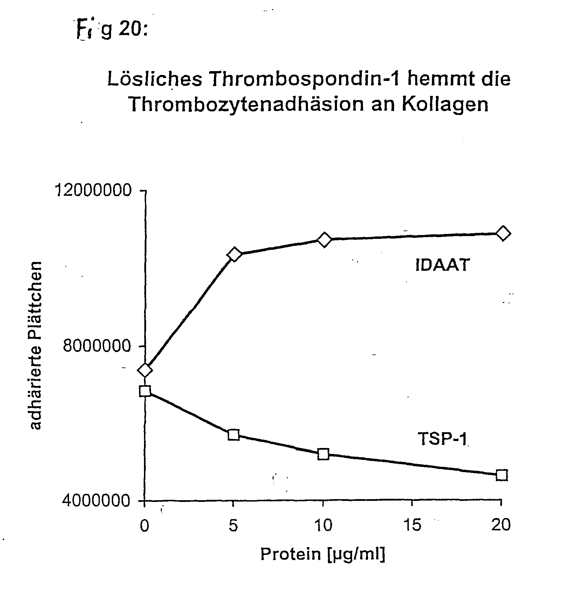

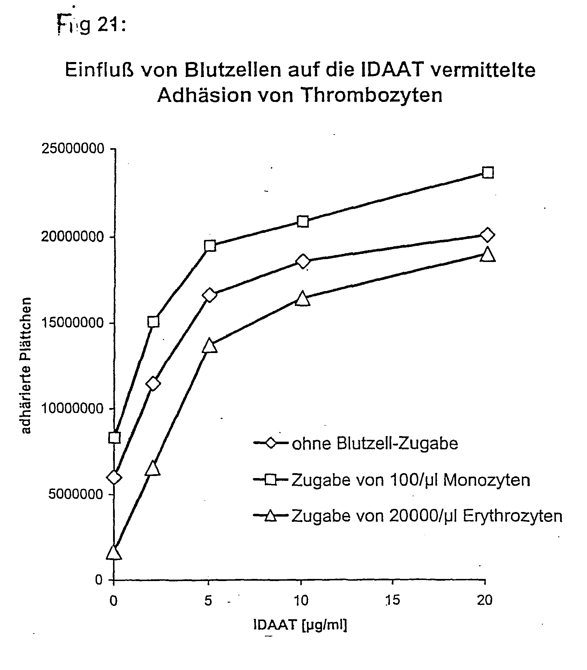

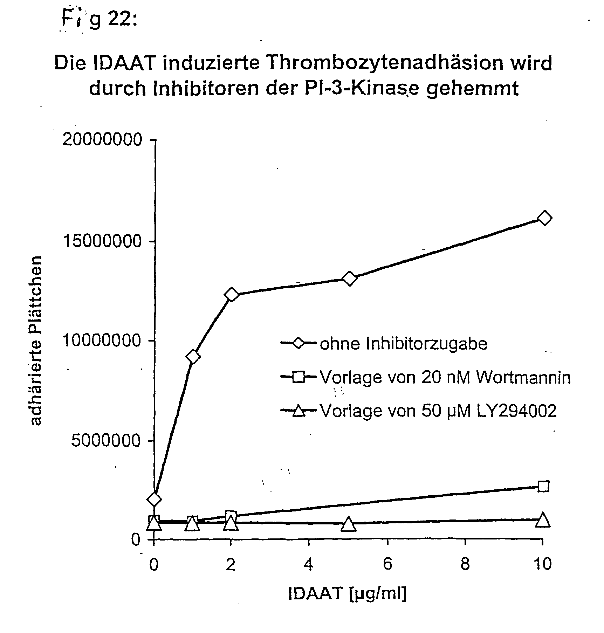

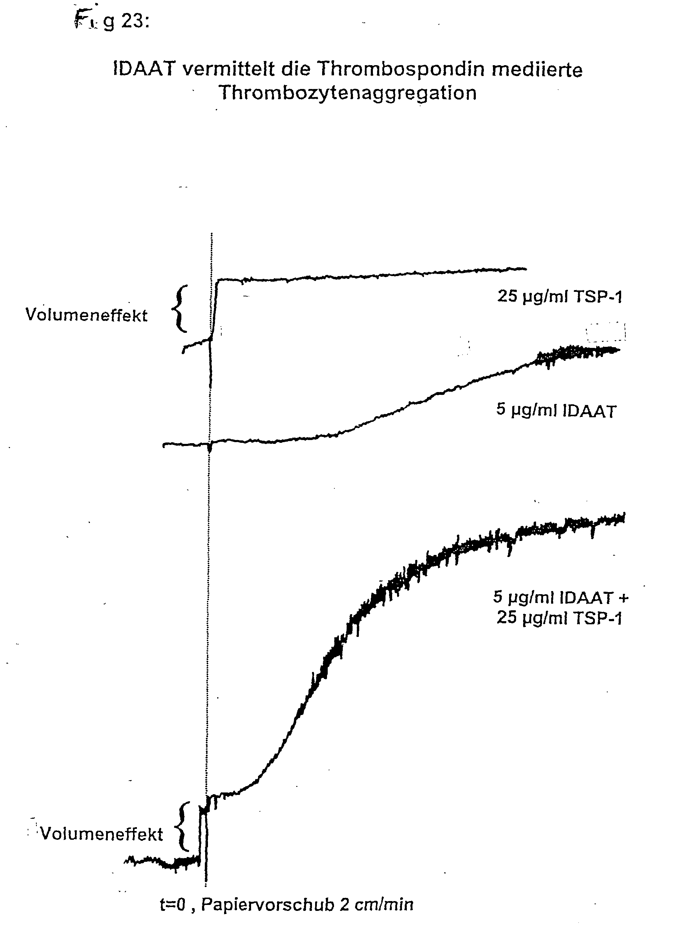

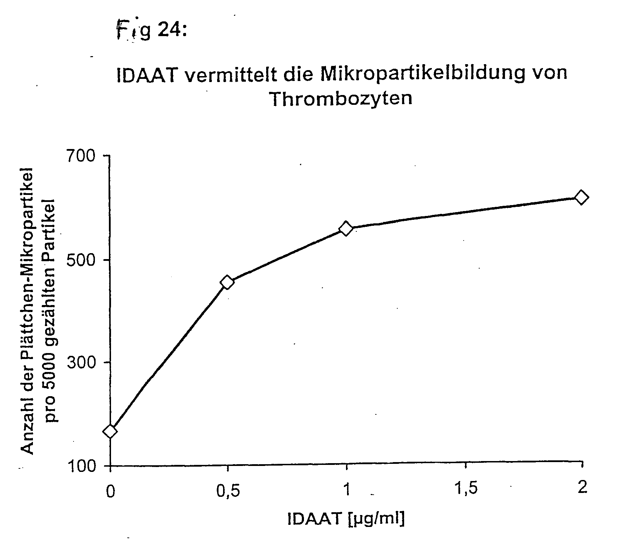

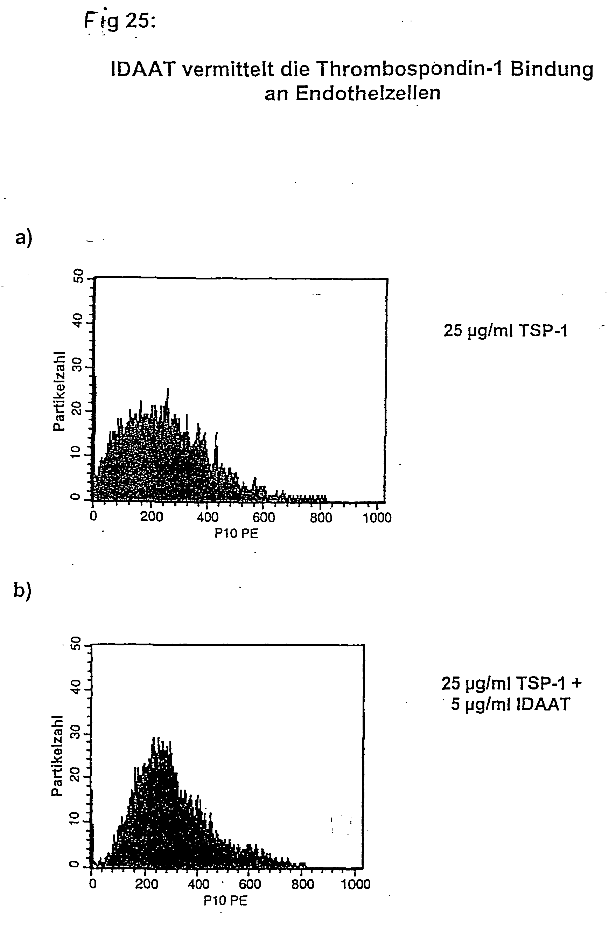

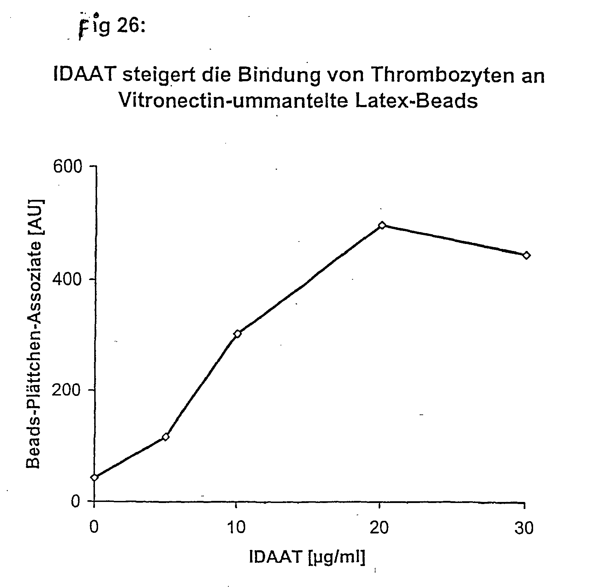

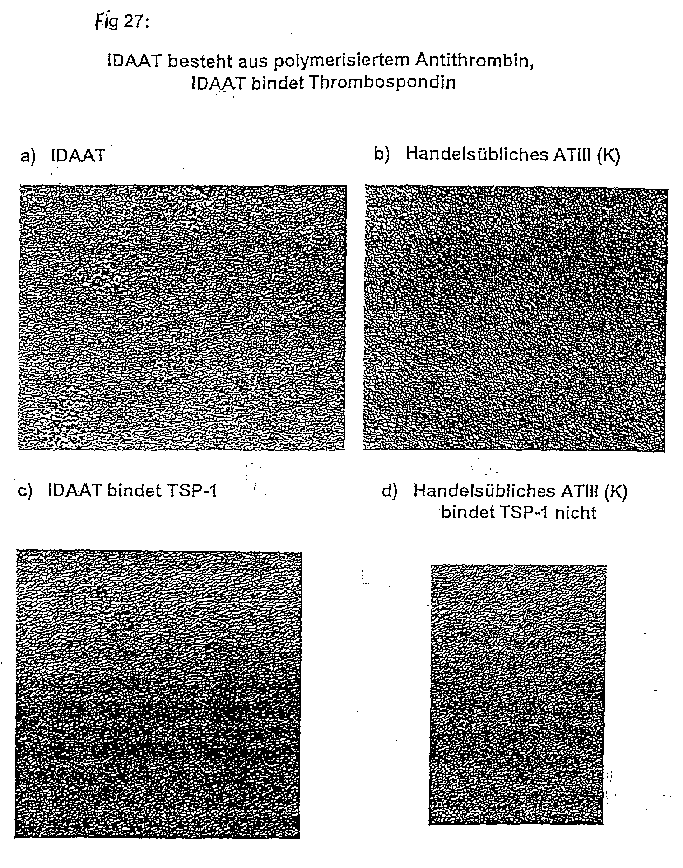

Durch die im Rahmen der vorliegenden Erfindung festgestellten Vorgänge im Körper, welche durch aktiviertes Antithrombin III bewirkt werden, kann das erfindungsgemäße Arzneimittel für eine Vielzahl von Indikationen angewandt werden. Im Folgenden sollen Beispiele für neue Funktionen von IDAAT aufgeführt werden, die Antithrombin-Präparate und insbesondere handelsübliche Antithrombin-Präparate nicht aufweisen:
1) IDAAT vermittelt spezifisch und dosisabhängig die Bindung von TSP-1 an Monozyten, monozytären Zelllinien und monozytären Zellen, wie Makrophagen.
   Während ohne Zusatz von gereinigtem TSP-1 und ohne Zusatz von IDAAT nur ~ 1 % der elutriierten humanen Monozyten durch einen Antikörper (Klon P10), der TSP-1 auf der Zelloberfläche erkennt, im Durchflußzytometer detektiert werden konnten, steigt die Anzahl durch Zusatz von 10 *µ*g/ml gereinigtem TSP-1 auf ca. 5%.
   Zusatz von IDAAT (ohne Zusatz von gereinigtem TSP-1) vermittelt die TSP-1 Bindung von endogenem TSP an die Monozyten. Ca. 18% der Monozyten wurden TSP-1 positiv.
   Durch gleichzeitige Gabe von TSP und IDAAT wurden nahezu alle (> 90%) der verwendeten periphären Blutmonozyten stark positiv für TSP (siehe Abbildung 1).
2) IDAAT vermittelt die TSP Anbindung an apoptotische PMNL
   Durch Alterung (24 h Inkubation in Zellkulturmedium im Inkubator gemäß Savill 1992) apoptotisch gemachte PMNL binden TSP. Dieser Vorgang wird durch Zusatz von IDAAT dosisabhängig, spezifisch gesteigert (siehe Abbildung 2). Gleichzeitige Zugabe von gereinigtem TSP + IDAAT steigert die Wirkung weiter.
3) IDAAT quervernetzt über TSP apoptotische PMNL mit Monozyten Zusatz von TSP und IDAAT führt dosisabhängig zur Assoziation von apoptotischen PMNL mit Monozyten (siehe Abbildung 3).
4) IDAAT fördert dosisabhängig die Transmigration von Monozyten durch Endothel
   Transmigrationsexperimente wurden, wie bei Kielbassa et al., 1998 beschrieben, durchgeführt. Die Zugabe von IDAAT zum Kulturmedium der Monozyten während eines Transmigrationsversuchs fördert dosisabhängig die Transmigration von Monozyten durch Endothel um das 2-3fache (siehe Abbildung 4).
   Auch der Zusatz von gereinigtem TSP (25 *µ*g/ml) fördert die Transmigration der Monozyten. Zusatz von TSP + IDAAT führt bei geringen Konzentrationen von TSP und IDAAT zu einer Steigerung der Transmigration, die größer ist, als durch Zusatz der Einzelsubstanzen alleine.
5) IDAAT aktiviert Monozyten
   IDAAT induziert dosisabhängig Ca²⁺-Flux in Monozyten. Die Ca²⁺-Messung wurden nach Sorrani et al., 1993 durchgeführt. Elutriierte Monozyten (5x10⁶/ml) wurden bei Raumtemperatur mit Hepes-Tyrode Puffer pH 7.4 gewaschen und anschließend 15 Min mit 1 µM Fura2/AM bei 37 °C markiert, zweimal in Hepes-Tyrode Puffer ohne Ca²⁺ gewaschen und dann in Hepes-Tyrode mit 1 mM Ca²⁺ aufgenommen.
   Ca²⁺-Signale induziert durch IDAAT, ⁻TSP-Peptid RFYVVMWK, und als positiv oder negativ Kontrollen wirksame Substanzen, wurden fluorimetrisch im Hitachi F-2000 bestimmt.
   IDAAT (100 *µ*g/ml) aktiviert die Monozyten und ruft ein deutliches Ca²⁺-Signal hervor (siehe Abbildung 5).
6) IDAAT vermittelt die Anbindung von TSP-1 an T-Zellen und an dendridische Zellen
   IDAAT vermittelt die Anbindung von T-Zellen sezerniertem TSP und von exogen zugesetztem TSP an humane T-Zellen (hier als Beispiel Jurkatzellen) (siehe Abbildung 6).
7) IDAAT verstärkt dosisabhängig die aktivierende Wirkung von fMLF auf den oxidativen Burst von PMNL
   Die Induktion des oxidativen Burst wurde im wesentlichen gemäß Arbeitsanleitung des Herstellers mit dem Phagotest/Burst-Test der Firma Orpegen (Heidelberg) am Durchflußzytometer durchgeführt, jedoch zuerst die PMNL mit dem Substrat DHR123 inkubiert und anschließend die PMNL aktiviert.
   IDAAT steigert dabei dosisabhängig die aktivierende Wirkung von fLMF auf den oxidativen Burst. IDAAT und fLMF haben beide eine additive Wirkung. IDAAT steigert nicht nur die aktivierende Wirkung von anderen Agonisten auf den oxidativen Burst der PMNL, sondern löst ihn als selbständiger, unabhängiger Agonist auch aus (siehe Abbildung 7). Somit ist IDAAT ein wertvolles Werkzeug zur Steigerung der Infektabwehr.
8) IDAAT inhibiert spezifisch und dosisabhängig die Freisetzung von aktivem Interleukin 12 (IL-12) durch aktivierte Monozyten
   Aktives IL-12 spielt eine negative Schlüsselrolle bei Entzündungsreaktionen und Sepsis. Mit Interferon γ (INF γ) und Staphylococcus aureus aktivierte Monozyten produzieren IL-12 und setzen es frei.
   Diese Reaktion konnte durch IDAAT dosisabhängig gehemmt werden. Die Konzentration von aktivem IL-12 im Kulturmedium der Monozyten wurde mittels ELISA bestimmt.
   Durch Inkubation der Monozyten mit IDAAT wurde die IL-12 Freisetzung vollständig inhibiert (siehe Abbildung 8).
   Im Gegensatz zur Sekretion von IL-12 wird die Sekretion von IL-10, welches in der Sepsis schützende Wirkung hat, durch IDAAT dosisabhängig gesteigert (siehe Abbildung_9). Dies zeigt die Infekt-Abwehr modulierende Wirkung von IDAAT und legt die Nützlichkeit von IDAAT bei septischen Reaktionen nahe. Die Freisetzung eines weiteren "schädlichen" Interleukins, des TNF α, wird durch IDAAT inhibiert (Abbildung 10).
9) IDAAT inhibiert Entzündungsreaktionen in vivo
   Im Ohr von Balb-C-Mäusen wurde durch lokale Injektion von anti-BSA zum Zeitpunkt 0 und gleichzeitiger Injektion von FITC-gekoppeltem BSA ins Peritoneum eine Arthus Reaktion ausgelöst. Kontrolltieren (Negativkontrollen) wurde nur FITC (ohne BSA) ins Peritoneum injiziert. Nach ca. 6 Stunden zeigte sich eine deutlich ausgeprägte Entzündungsreaktion mit Anschwellung des Ohres (Oedem), FITC-Einlagerung, Einwanderung von PMNL und petechialen Einblutungen ins Gewebe bei den mit anti BSA- und BSA-FITC behandelten Tieren.
   8 Mäusen wurde zusätzlich zum Zeitpunkt 0 und nach 0 + 3 Stunden je 50 µg IDAAT in Puffer intraperitonal injiziert.
   6 Kontrollmäusen erhielten zum Zeitpunkt 0 und 0 + 3 Stunden statt IDAAT nur den Puffer, 50 mM Tris/HCl-Puffer mit 150 mM NaCl pH 7.4, in dem IDAAT üblicherweise gelöst wird.
   Die Arthus Reaktion wurde durch IDAAT fast vollständig verhindert. Mit IDAAT behandelte Mäuse zeigten signifikant geringere FITC-Einlagerungen, signifikant geringere Ohrdicken und fast keine Petechien im Vergleich zu "Puffer" behandelten Tieren (siehe Abbildung 11).
10) IDAAT inhibiert die HIV-1 Infektion von monozytären Zellen aus periphärem Blut (PBMC)
   PHA-aktivierte PBMC wurden zusammen mit negativem humanen Serum 1:100 (Negativ-Kontrolle), mit neutralisierendem V3loop spezifischen Antikörper (Positivkontrolle), mit IDAAT (150 µg/ml) und einem CCR5-tropen HIV-1 Primärisolat (903) aus einem Patienten inkubiert und nach fünf Tagen die Virus-Produktion mittels p24-ELISA untersucht.
   Dazu wurden frisch PHA-aktivierte PBMC in RPMI 1640 Medium + 20% FKS + 100 U/ml IL-2 in einer Zellkonzentration von 2x10⁶ Zellen/ml aufgenommen und mit jeweils 200.000 Zellen/well/100 *µ*l auf einer 96well-Flachbodenplatte verteilt.
   Auf Inhibition zu testende Substanzen,
   Positivkontrolle: neutralisierender humaner anti V3loop Antikörper 1:100, Negativkontrolle: neg. humanes Serum 1:100 und Verum: IDAAT (150 *µ*g/ml),
   wurden in RPMI-Medium den Zellen zugesetzt und für 30 Minuten bei 37 °C/5% CO₂ inkubiert. Anschließend wurde das HIV-1 Virus zu den Ansätzen gegeben: jeweils 10 *µ*l/well aus HIV-1 Primärisolat 903-Überstand (CCR5-trop) mit 20000 TCID₅₀ (50% tissue culture infective dose)/ml
   ≅ 1000 TCID₅₀/ml im well.
   Diese Ansätze wurden über Nacht bei 37°C/5%CO₂ inkubiert. Am nächsten Tag wurden die Zellen dreimal mit RPMI 1640 gewaschen und neues Kulturmedium zugegeben. Am 5. Tag nach der Infektion wurden die p24-ELISA-Untersuchungen durchgeführt.
   P24-ELISA:
   Die verwendeten α-p24-Antikörper (11-G7 [Niedrig, Berlin] und D7320 [Biochrom]) erkennen das p24-Protein der verwendeten Primärisolatvariante 903. Maxi-Sorb-ELISA-Platten (Nunc) wurden mit diesen Antikörpern über Nacht beschichtet. Der Virus-Überstand aus dem Inhibitionsversuch wurde mit 1 % Triton X-100 inaktiviert. Der inaktivierte Virus-Überstand und der alkalische-Phosphatase-konjugierte- Detektions-Antikörper (BC1071-AP [Aalto]) wurden, nach Waschen der beschichteten Wells mit PBS, gemeinsam in die Wells überführt und dort für 5 Stunden bei 37°C inkubiert. Die Wells wurden wieder mit PBS gewaschen, gelöstes Substrat für die alkalische Phosphatase p-Nitrophenyl-Phosphat [Sigma] in die Wells gegeben und die Farbentwicklung nach 20 Minuten bei 405 nm im ELISA-Photometer gemessen. Die Parallelwerte in dem verwendeten p24-ELISA schwankten bis zu 0.02 optische Dichte (oD) Einheiten um einen gemeinsamen Mittelwert.
   Während die oD 405 nm für die Negativkontrolle ≅ keine Inhibition bei 0.8 lag, reduzierte der neutralisierende Antikörper (Positivkontrolle) die oD auf 0.12. 150 *µ*g/ml IDAAT reduzierte die oD auf 0.10.
   Durch Zugabe von IDAAT konnte die HIV-1 Infektion der PBMCs effektiv gehemmt werden.
11) IDAAT vermittelt die S. aureus-Bindung an zur Phagozytose und Bakterienabwehr fähige Zellen (Blutplättchen, Monozyten, PMNL)
   Thrombozyten wurden mit einem Thrombozytenspezifischen Phycoerythrin konjugierten anti GPIX Antikörper (Klon Beb 1) markiert. Bakterien (verschiedene S.aureus Stämme) wurden mit Tris-gepufferte Salzlösungen (TBS) auf eine Zahl von 250.000 Keime/µl eingestellt und mittels RNA-Farbstoff Syto 13 [MoBiTec, Göttingen] in einer Konzentration von 2 µM markiert. Markierte Bakterien und markierte Thrombozyten wurden im Verhältnis von 10:1 für 10 Minuten koinkubiert.
   Zellpopulationen wurden im Durchflußzytometer analysiert. Für beide Fluorochrome positive Zellen wurden als Assoziate gewertet (siehe Abbildung 12 a). Thrombin-Stimulierung und Freisetzung von TSP aus den α-Granula der Plättchen erhöhte den prozentualen Anteil der bakterientragenden Plättchen im Verhältnis zur Thrombozytengesamtzahl um das 2.5fache. Diese Steigerung war nicht zu beobachten bei Verwendung von Plättchen von 2 Patienten mit Gray-Platelet-Syndrom, deren Plättchen das TSP fehlt (siehe Abbildung 12 b).
   IDAAT förderte dosisabhängig die Bindung von S. aureus an Thrombozyten (siehe Abbildung 12 c).
   Da Thrombozyten ein sogenanntes "microbicidal protein" haben, welches Bakterien vernichten kann, ist auch diese Funktion von IDAAT als wertvoller Beitrag zur Infektabwehr zu werten.
12) IDAAT verbessert die Blutstillung
   a) IDAAT vermittelt die Bindung von Thrombospondin an Thrombozyten Gereinigtes TSP-1 wurde mit FITC markiert und in einer Konzentration von 50 *µ*g/ml gelfiltrierten Thrombozyten (50.000/*µ*l) in Hepes-Tyrode Puffer mit BSA zugesetzt. IDAAT wurde in steigenden Konzentrationen zugesetzt und 60 Minuten zusammen mit den Plättchen bei Raumtemperatur inkubiert. Gebundenes TSP-FITC auf der Thrombozytenoberfläche wurde im Durchflußzytometer quantifiziert. IDAAT vermittelte die Bindung von Thrombospondin an Thrombozyten (siehe Abbildung 13).
   b) IDAAT fördert die Fibrinogen-Bindung an Thrombozyten Gelfiltrierten Plättchen 50.000/µl in Hepes- Tyrode BSA Puffer oder mit PPACK antikoaguliertes Plättchenreiches Plasma (50.000/µl) wurde FITCkonjugiertes Fibrinogen (150 µg/ml) zugesetzt. Die Plättchensuspensionen wurden mit meth. Kollagen Typ I, wie in Kehrel et al., 1998 beschrieben, aktiviert. Ein Teil der Proben wurde mit IDAAT in aufsteigenden Konzentrationen versetzt. Nach Inkubation für 30 Minuten bei Raumtemperatur wurden die Plättchen fixiert, gewaschen und die Fibrinogenanbindung im Durchflußzytometer quantitativ bestimmt. IDAAT verstärkt die durch Kollagenaktivierung induzierte Fibrinogenanbindung an Plättchen (siehe Abbildung 14a).
      Zusatz von gereinigtem Thrombospondin + IDAAT in steigenden Konzentrationen hatte selbst eine Plättchen-aktivierende Eigenschaft und führte zu Fibrinogenbindung an die Plättchenmembran (siehe Abbildung 14b).
   c) IDAAT fördert die Adhäsion von Thrombozyten an Adhäsionsproteine wie Thrombospondin, Fibrinogen, Fibronectin, Vitronectin und Kollagen.
      Die Adhäsion der Thrombozyten wurde nach Santoro et al., 1994 durchgeführt. Mikrotiterplatten (96 well) wurden mit Adhäsionsproteinen in einer Konzentration von 25 *µ*g/ml über Nacht bei 4°C "gecoatet" und die Platten mit BSA geblockt. 100 *µ*l gelfiltrierten Plättchen oder mit Hirudin antikoaguliertes Plättchenreiches Plasma (300.000 Plt/*µ*l) wurden bei Raumtemperatur für 1 Stunde in einer feuchten Kammer in den Wells inkubiert. Nicht adhärierte Thrombozyten wurden gründlich ausgewaschen. Die Anzahl der adhärierten Plättchen wurde durch Lyse der Plättchen mit Triton X-100 und Nachweis des lysosomalen Enzyms Hexosaminidase bestimmt.
      Zur Kalibrierung des Adhäsions-Assays wurde auf der Mikrotiterplatte ein Eichreihe mit bekannter Anzahl ansteigender Plättchen gegeben und die Extinktion des umgesetzten Substrates P-Nitrophenyl-N-Acetyl-β -D-Glucosaminid im Verhältnis zur Plättchenzahl bestimmt. IDAAT steigerte dosisabhängig die Adhäsion von Thrombozyten an die getesteten Adhäsionsproteine (siehe Abbildung 15a). Handelsübliche ATIII-Präparate zeigten diese Wirkung nicht (siehe Abbildung 15b).
      Die Steigerung der Thrombozytenadhäsion durch IDAAT ist eine Integrin (αᵥβ₃*,* αllbβ3)-und CD36-vermittelte Reaktion (siehe Abbildung 16 und 17).
      Die IDAAT vermittelte Thrombozytenadhäsion ist nicht Thrombin-vermittelt und findet daher auch im mit Hirudin antikoaguliertem Blut statt (siehe Abbildung 18).
      Heparansulfat (0-10 µg/ml) und Sulfatid (0-20 µg/ml) hemmen die durch IDAAT vermittelte Adhäsion nicht.
      Die IDAAT vermittelte Thrombozytenadhäsion ist abhängig von zweiwertigen Ionen. 5 mM EDTA hemmen diese Adhäsion an Thrombospondin und an Kollagen vollständig (siehe Abbildung 19). 20 µM Mg²⁺, 1 mM Ca²⁺ oder andere zweiwertige Ionen steigern die IDAAT vermittelte Thrombozytenadhäsion an Kollagen.
      Lösliches TSP-1 hemmt die IDAAT vermittelte Adhäsion von Thrombozyten an Kollagen oder immobilisiertes TSP-1 (siehe Abbildung 20).
      Zusatz von Monozyten zu den Thrombozyten steigert die Adhäsion der Thrombozyten an Thrombospondin und an Kollagen, während der Zusatz von Erythrozyten die Adhäsion von Thrombozyten an TSP und an Kollagen hemmt (siehe Abbildung 21).
      Die IDAAT vermittelte Adhäsion von Thrombozyten an Thrombospondin kann durch die PI-3-Kinase-Hemmer Wortmannin und LY294002 vollständig gehemmt werden (siehe Abbildung 22).
   d) IDAAT vermittelt die TSP-medüerte Aggregation von Thrombozyten Gelfiltrierte Plättchen (200.000/µl) in Hepes- Tyrode Puffer pH7.4 mit Zusatz von Fibrinogen (100 µg/ml) wurden im Aggregometer nach Born untersucht. Während gereinigtes Thrombospondin (25 µg/ml) allein keine Aggregation auslöste, führte der Zusatz von IDAAT dosisabhängig zur Aggregation, die durch gleichzeitige Gabe von TSP und IDAAT deutlich verstärkt wurde (siehe Abbildung 23).
   e) IDAAT vermittelt die Mikropartikelbildung von Thrombozyten Gelfiltrierte Plättchen wurden mit dem TSP-1 Peptid RFYVVMWK (40*µ*M) aktiviert. Die Mikropartikelbildung wurde nach Markierung mit einem Thrombozyten-spezifischen anti GPIX Phycoerytrin konjugierten Antikörper nach Dörmann et al., 1999, im Durchflußzytometer gemessen. Zusatz von IDAAT führte dosisabhängig zur Mikropartikelbildung der aktivierten Plättchen (sie Abbildung 24).
   f) IDAAT vermittelt die Assoziation von Thrombozyten und Leukozyten Zur durchflußzytometrischen Detektion der Plättchen-Leukozyten-Assoziate wurden die Thrombozyten mit einem FITC-konjugierten monoklonalen Antikörper gegen das Plättchen-spezifische Antigen GPIX (Klon Beb 1) und die Monozyten mit einem PE-konjugierten monoklonalen Antikörper gegen CD14 (Klon: MΦP9) in sättigenden Konzentrationen nach Zellaktivierung und Zellfixierung markiert. Die Quantifizierung der Assoziate erfolgte durch Detektion von CD14-und GPIX-positiven Partikeln. Der prozentuale Anteil von Leukozyten, die mit Plättchen assoziiert vorlagen, wurde zur Leukozytengesamtpopulation ins Verhältnis gesetzt. Thrombozyten (25.000/µl) und Monozyten (3000/µl) wurden miteinander für 30 Minuten inkubiert. Die Thrombozyten wurden zuvor für 30 Minuten mit IDAAT vorinkubiert und anschließend gewaschen. IDAAT steigerte die Assoziationsrate von 11.7% auf 17,3% (5*µ*g/ml IDAAT) bzw. 20.5% (10 mg/ml IDAAT).
13) IDAAT vermittelt/verstärkt die TSP-Anbindung an Endothelzellen
   Humane mikrovaskuläre Endothelzellen (HMEC-1) (3000/µl) in RPMI 1640 Medium wurden für 30 Minuten bei Raumtemperatur mit IDAAT oder TSP-1 (25 µg/ml) plus IDAAT inkubiert. Die Endothelzellen wurden fixiert, gewaschen und TSP mit einem monoklonalen PE-konjugierten anti TSP-Antikörper (Klon P10) im Durchflußzytometer detektiert. Der Median der Fluoreszenz, der die Anbindung des anti TSP-Antikörpers widerspiegelt, stieg von 79 (ohne IDAAT) auf 138 (5 *µ*g/ml IDAAT). Zusatz von exogenem TSP-1 (25 *µ*g/ml) steigerte den Median der Fluoreszenz auf 268 bei gleichzeitigem IDAAT-Zusatz (5 *µ*g/ml) (siehe Abbildung 25).
14) IDAAT vermittelt die Bindung von Thrombozyten an mit Vitronectin ummantelte Latex-"Beads"
   Latex Beads (3.2 *µ*m) wurden über Nacht bei 4°C mit aktivem Vitronectin (25 *µ*g/ml) ummantelt und dann gewaschen. Vitronectin-tragende Beads wurden mit gelfiltrierten Thrombozyten (25.000/*µ*l) in Abwesenheit und Gegenwart von steigenden Konzentrationen an IDAAT für 1 Stunde bei Raumtemperatur inkubiert.
   Thrombozyten wurden mit anti GPIX (Klon Beb1) markiert und Assoziate aus Vitronectin ummantelten Beads mit Thrombozyten im Durchflußzytometer quantifiziert. IDAAT steigerte dosisabhängig die Bindung von Thrombozyten an die Vitronectin ummantelten Beads (siehe Abbildung 26).
15) IDAAT besteht aus polymerisiertem ATIII
   IDAAT, IDAAT-TSP-1-Aggregate, handelsübliches ATIII und handelsüliches ATIII mit TSP-1-Zusatz wurden elektronenmikroskopisch mittels "Rotary-Shadowing"-Verfahren nach Jander et al (1984) dargestellt. IDAAT bestand aus polymeren ATIII-Molekülen, während handelsübliche ATIII-Präparate eine monomere Struktur aufwiesen (siehe Abbildungen 27 a und b). Zusatz von TSP-1 zu IDAAT führte zur Bildung von großen IDAAT-TSP-1-Komplexen, während dies nicht mit handelsüblichem ATIII beobachtet wurde (siehe Abbildungen 27 c und d).
16) IDAAT erhält neue proteinbindende Eigenschaften

IDAAT bindet direkt an Proteine, an die nichtaktiviertes Antithrombin nicht binden kann, wie CD4 (z.B. der T-Zellen) (Abbildung 28), GP120 des HI-Virus (Abbildung 29), Thrombospondin (Abbildung 30), aktives Vitronectin (Abbildung 31), CD36, α*ᵥ*β3-Integrin. Die Bindung von IDAAT an diese Proteine wurde mittels ELISA an gereinigten oder rekombinanten Proteinen durchgeführt. Die Reinigung von TSP-1, aktivem Vitronectin, α_{IIb}β3-Integrin und CD36 erfolgte wie bei Kehrel et al. 1993, Yatohgo et al. 1988 und Kronenberg, Grahl und Kehrel 1998, beschrieben.

Die Herstellung von IDAAT kann im Rahmen der vorliegenden Erfindung beispielsweise wie folgt erfolgen:
Handelsübliches Antithrombin III wird mit NaOCl oxidiert und über eine Sephadex-Säule gegeben (Beispiel 1). Vor der Oxidation kann Antithrombin III vorzugsweise noch mit neutrophiler Granulozyten-Elastase inkubiert werden (siehe Beispiel 2). Vor der Oxidation kann Antithrombin III auch mit Matrixmetalloproteinase gespalten werden (siehe Beispiel 3). Antithrombin III kann auch durch Reaktion mit Defensin 2 aktiviert werden (siehe Beispiel 4).

Dieses Verfahren zur Herstellung von IDAAT ist ein weiterer Gegenstand der vorliegenden Erfindung.

Wiederum ein weiterer Gegenstand der vorliegenden Erfindung ist die Verwendung eines erfindungsgemäßen Arzneimittels, wobei die im Folgenden genannten Krankheiten oder Krankheitszustände bei Menschen oder Tieren behandelt werden können. Das erfindungsgemäße Arzneimittel kann dabei sowohl zur Prophylaxe als auch als Heilmittel eingesetzt werden. Indikationen sind akute Infektionen, insbesondere Infektionen mit Erregern, die direkt oder indirekt an IDAAT oder einen Interaktionspartner binden, ganz besonders: die Gruppe der HI-Viren, Parasiten wie Plasmodium falciparum und Pneumocystis carinii, und Bakterien wie z.B. Staphylococcus aureus; Verbesserung der lmmunabwehr und als Mittel zur Prophylaxe der Sepsis bei Patienten mit hohem Infektionsrisiko wie z.B. nach Operation mit großer Infektionsgefahr, Polytraumen, Verbrennungen, Intoxikationen, unter Chemotherapie, bei immunsupprimierten Patienten und Patienten mit prädisponierter Abwehrschwäche; zur medikamentösen Beeinflussung von akuten, chronischen oder allergischen Entzündungsreaktionen, insbesondere zur Modulation von Entzündungsreaktionen, bei denen apoptotische PMNL und Eosinophile Granulozyten unschädlich gemacht werden sollen; als Heilmittel zur Bekämpfung von Tumorwachstum und -metastasieung; als Angiogenesehemmer zur Bekämpfung von unerwünschter Neoangiogenese, z.B. in Tumoren oder Patienten mit Retinopathie; als Heilmittel für Leukämie, insbesondere chronisch lymphatische Leukämie; zur Verbesserung der Wundheilung, insbesondere bei schlecht heilenden Wunden und in der plastischen Chirurgie; zur Behandlung von Läsionen im Nervensystem, insbesondere bei Erkrankungen, bei denen ein Auswachsen von Neuriten erwünscht ist; zur Verbesserung der Blutstillung, insbesondere bei Patienten mit angeborenen oder erworbenen Blutgerinnungsstörungen oder angeborenen oder erworbenen Thrombozytopathien, unter Antikoagulationstherapie, oder bei Operationen unter Herz-Lungenmaschine; zur Verhinderung von entzündlich bedingten Gewebeschädigungen, z.B. bei a) Reperfusionen (z.B. Schlaganfall, Myokardinfarkt, Abschnürungen)
b) Organtränsplantationen (Verhinderung der Transplantatabstoßung) und
c) allergischen Reaktionen (incl. Neurodermitis, Asthma bronchiale).

Wenn das erfindungsgemäße Arzneimittel zur Prophylaxe von Erkrankungen eingesetzt wird, kann es allein oder in Kombination mit Interaktionspartriern oder anderen Medikamenten auch in Spülflüssigkeiten als Zusatz z.B. für Mund, Vagina, Anus und Augen, als Zusatz zur Vorkehrung gegen Übertragung von Infektionen bei sexuellen Kontakten (wie z.B. Kondomen, Diaphragmen etc.) und in Lösungen, Pflastern und Wundauflagen zur Wundpflege angewandt werden.

Die Anwendung des erfindungsgemäßen Arzneimittels ist lokal, intradermal, oberflächlich, intraperitonal, intravenös, intramuskulär, oral oder über Vesikel in einer der oben genannten Applikationsformen denkbar.

Eventuelle andere Applikationsarten sind ebenfalls von der vorliegenden Erfindung umfasst.

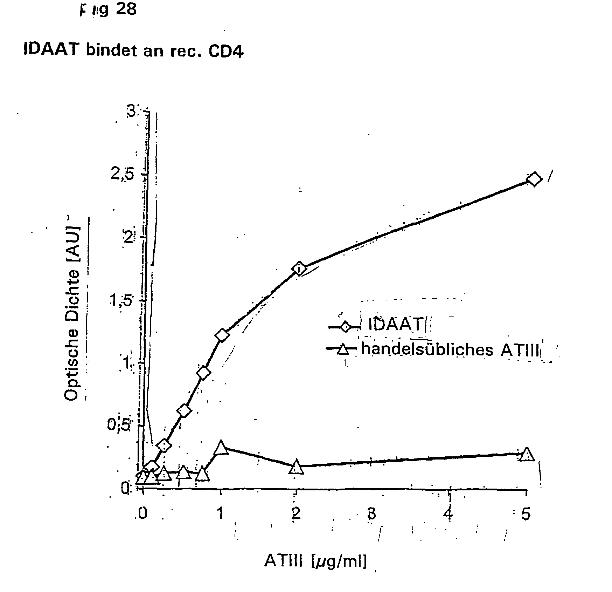

Die vorliegende Erfindung wird im Folgenden noch durch die Abbildungen und die Beschreibungen dazu erläutert:
**Abbildung 1**: IDAAT vermittelt die TSP-1 Bindung an Monozyten
   TSP-1 positive Monozyten wurden mit dem monoklonalen anti TSP Antikörper Klon P10, der mit Phycoerythrin konjugiert war, markiert und die Fluoreszenz der Monozyten im Durchflußzytometer gemessen
   a) 3000 gemessene Monozyten: Monozyten mit Hepes-Tyrode Puffer pH 7.4 30 min inkubiert; gewaschen und mit P10-PE markiert
   b) 3000 gemessene Monozyten: Monozyten mit TSP-1 (10 *µ*g/ml) in Hepes-Tyrode Puffer pH7.4 30 min, RT inkubiert; gewaschen und mit P10-PE markiert
   c) 3000 gemessene Monozyten: Monozyten mit TSP-1 (10 µg/ml) und IDAAT (10 µg/ml) in Hepes-Tyrode Puffer pH7.4 30 min, RT inkubiert; gewaschen und mit P10-PE markiert
   d) Monozyten mit und ohne Zusatz von TSP-1 (10*µ*g/ml) mit IDAAT in steigenden Konzentrationen inkubiert (s.o.); IDAAT vermittelt die Bindung von exogen zugesetztem und endogen vorhandenem TSP-1
**Abbildung 2:** IDAAT vermittelt die TSP-1 Bindung an apoptotische polymorphkernige Granulozyten (PMNL)
   a) PMNL wurden durch Inkubation in Zellkulturmedium für 24 Stunden im Inkubator nach Savill et al., 1992 apoptotisch gemacht. Apoptotische PMNL wurden mit Hepes-Tyrode Puffer pH7.4 30 min inkubiert, gewaschen, mit anti TSP AK P10-PE markiert und die Fluoreszenz von 3000 Zellen durchflußzytometrisch gemessen
   b) PMNL wurden durch Inkubation in Zellkulturmedium für 24 Stunden im Inkubator nach Savill et al., 1992 apoptotisch gemacht. Apoptotische PMNL wurden mit TSP-1 (5 *µ*g/ml) und IDAAT (10 *µ*g/ml) in Hepes-Tyrode Puffer pH7.4 30 min inkubiert, gewaschen, mit TSP AK P10-PE markiert und die Fluoreszenz von 3000 Zellen durchflußzytometrisch gemessen.
   c) Durchführung analog a und b: IDAAT in steigenden Konzentrationen eingesetzt. IDAAT vermittelt die Bindung von endogen vorhandenem und exogen zugesetztem (10 *µ*g/ml) Thrombospondin.
**Abbildung 3:** IDAAT quervernetzt apoptotische PMNL mit Monozyten durch TSP
   Elutriierte PMNL und elutriierte Monozyten wurden mit TSP und IDAAT in unterschiedlichen Konzentrationen für 30 min bei RT miteinander inkubiert und die Zellen fixiert. Die PMNL wurden mit einem monoklonalen FITC-konjugierten Antikörper gegen CD 16b und die Monozyten mit anti CD 14-PE markiert. PE und FITC positive Assoziate wurden mit dem Durchflußzytometer gemessen.
**Abbildung 4:** In "Transwell"-Zellkultur-Kammern (Costar, Bodenheim) wurde je ein mit einer mikroporösen Polycarbonat-Membran bespannter "Transwell"-Einsatz pro einer der 24-Vertiefungen plaziert. Die Polycarbonat-Membran mit einer Porengröße von 5 *µ*m wurde mit Fibronectin beschichtet und darauf humane mikrovaskuläre Endothelzellen (HMEC-1) bis zur Konfluenz kultiviert.
   Durch Dichtegradientenzentrifugation isolierte humane Monozyten (200 *µ*l mit 2x10⁷ Zellen/ml in DMEM aus dem periphären Blut) wurden nach einem Tag in Kultur in den oberen "Transwell"-Einsatz gegeben und für 4 Stunden bei 37°C, 7%CO₂ mit dem HMEC-1 Monolayer inkubiert. Als Maß für die Transmigrationsrate wurde die Anzahl der Monozyten im unteren "Transwell"-Kompartiment unter dem Transwell-Einsatz bestimmt.
   Um den Einfluß von verschiedenen ATIII-Präparaten auf die Transmigrationsrate der Monozyten zu untersuchen, wurden entweder Monozyten, oder Endothelzellen für 10 min mit den Testsubstanzen vorinkubiert und gewaschen oder die Testsubstanzen dem Medium in der oberen "Transwell"-Kammer zugesetzt und die Testsubstanzen während des kompletten Transmigrationsversuchs dort belassen. Hier ist der Zusatz zum Medium während der Transmigration dargestellt.
   Nach 4 Stunden Transmigrationsdauer wurden die Einsätze vorsichtig entfernt, die Zellkulturplatte für 30 min zur Ablösung der adhärierten Monozyten auf Eis gestellt und die Zahl der transmigrierten Monozyten ausgezählt.
**Abbildung 5:** IDAAT aktiviert Monozyten und ruft ein Ca²⁺-Signal hervor. Die angewandte Methodik wurde in der Beschreibung des Beispiels detailliert dargestellt.
**Abbildung 6:** IDAAT vermittelt die Anbindung von TSP-1 an T-Zellen Kultivierte humane T-Zellen (Jurkatzellen) wurden für 1 Stunde bei RT mit IDAAT oder IDAAT plus TSP-1 in den angegebenen Konzentrationen inkubiert. An die T-Zeflen gebundenes TSP-1 wurde durch den monoklonalen PE-konjugierten anti TSP Antikörper (Klon P10) markiert und im Durchflußzytometer, gemessen. IDAAT vermittelt die Bindung von endogen vorhandenem und exogen zugesetztem TSP an T-Zellen.
   a) ohne TSP-1 Zusatz; ohne IDAAT Zusatz; anti TSP-AK-PE-Markierung
   b) TSP-1 Zusatz (25 µg/ml); ohne IDAAT Zusatz; anti TSP-AK-PE-Markierung
   c) TSP-1 Zusatz (25 *µ*g/ml); IDAAT Zusatz (1 *µ*g/ml); anti TSP-AK-PE-Markierung
   d) TSP-1 Zusatz (25 *µ*g/ml); IDAAT Zusatz (5 *µ*g/ml); anti TSP-AK-PE-Markierung
   e) Plus/minus TSP-1 (25 *µ*g/ml); IDAAT in steigenden Konzentrationen; anti TSP-AK-PE-Markierung
**Abbildung 7:** IDAAT verstärkt die aktivierende Wirkung von fLMF auf den oxidativen Burst von PMNL
   a) fLMF löst dosisabhängig den oxidativen Burst der PMNL aus
   b) IDAAT steigert den durch fLMF ausgelösten oxidativen Burst und ist unabhängig von anderen Agonisten selbst in der Lage, den oxidativen Burst in PMNL zu induzieren
**Abbildung 8:** IDAAT inhibiert die Freisetzung von aktivem Interleukin 12 durch mit Interferon γ + S. aureus aktivierte Monozyten
   Die angewandte Methodik wurde in der Beschreibung des Beispiels detailliert dargestellt.
**Abbildung 9:** Die IL-10 Sekretion von mit S. aureus und Interferon γ aktivierten Monozyten wird durch IDAAT gesteigert. Damit fördert IDAAT die Sekretion eines Interleukins, welches im Tierexperiment vor LPS induzierter Lethalität schützt. Die angewandte_Methodik wurde analog Abbildung 8 durchgeführt. IL-10 wurde mittels ELISA bestimmt.
**Abbildung 10:** Die TNF α-Sekretion von mit S. aureus und Interferon γ aktivierten Monozyten wird durch das TSP-1 Peptid RFYVVMWK inhibiert (10a). IDAAT steigert die inhibierende Wirkung des TSP-1 Peptids (25 µM) (10b). TNF α wurde mittels ELISA bestimmt. Die angewandte Methodik wurde analog Abbildung 8 durchgeführt.
**Abbildung 11:** IDAAT inhibiert die Arthus Reaktion im Ohr von Balb-C Mäusen
   a) Zweimal zum Zeitpunkt 0 und 0+3 Stunden je 50 µg IDAAT i.p. behandelte Maus. Arthus Reaktion im linken Ohr
   b) Zweimal Kontrollpuffer i.p. behandelte Maus. Arthus Reaktion im linken Ohr
   c) In den Ohren eingelagertes BSA-FITC als Maß für die Arthus Reaktion in mit IDAAT oder Kontrollpuffer behandelten Mäusen. Arthus Reaktion im linken Ohr.
**Abbildung 12:**
   a) Dot-Plot Darstellung der Thrombozyten-Bakterien-Assoziation
      A: Thrombozyten markiert mit PE-konjugiertem anti GPIX Antikörper (Beb 1)
      B: Bakterien (S. aureus) markiert mit Syto 13
      C: Beide Fluoreszenzen emittierende Bakterien-Thrombozyten-Assoziate
   b) S. aureus (Cowan 1)-Thrombozyten-Assoziate
      Verwendung von Thrombozyten der Patienten A.P. und W.K. mit "Gray-platelet-syndrom". Die Zunahme der Assoziatrate durch Aktivierung mit Thrombin fehlt bei Verwendung von "Gray"-Plättchen, denen das Thrombospondin-1 fehlt.
      **p<0.005
      ***p<0.0001
   c) Die S. aureus (Cowan-1)-Thrombozyten-Assoziatbildung wird durch IDAAT gesteigert. Zusätzliche Zugabe von TSP-1 führt zu einer weiteren Steigerung der Anzahl der Assoziate.
**Abbildung 13:** Gereinigtes Ca2 +-haltiges TSP-1 aus humanen Thrombozyten wurde mit FITC (TSP-1-FITC) konjugiert und zu gelfiltrierten Plättchen gegeben. Dieser Ansatz wurde für 1 Stunde bei RT mit IDAAT (2 µg/ml und 5 µg/ml) inkubiert und im Durchflußzytometer gemessen (5000 Thrombozyten)
**Abbildung 14:**
   a) Gelfiltrierte humane Thrombozyten (50.000/*µ*l) wurden mit 150 *µ*/ml FITC-konjugiertem Fibrinogen versetzt und mit Kollagen in aufsteigenden Konzentrationen in Abwesenheit oder Anwesenheit von IDAAT (5 µg/ml) inkubiert. Nach 30 minütiger Inkubation wurden die Thrombozyten im Durchflußzytometer gemessen.
   b) Gelfiltrierte humane Thrombozyten (50.000/µl) wurden mit 150 µ/ml FITC-konjugiertem Fibrinogen versetzt und IDAAT ohne TSP oder mit TSP-1 (10 *µ*g/ml) in aufsteigenden Konzentrationen zugesetzt. Nach 1 stündiger Inkubation wurden die Thrombozyten im Durchflußzytometer gemessen.
**Abbildung 15:**
   a) IDAAT steigert die Adhäsion von Thrombozyten an Adhäsionsproteine: Fibronectin, Vitronectin, Fibrinogen, Thrombospondin-1 und Kollagen
   b) Vergleich von IDAAT und handelsüblichen ATIII-Präparaten auf die Wirkung der Plättchenadhäsion. Die angewandte Methodik wurde in der Beschreibung des Beispiels detailliert dargestellt.
**Abbildung 16:**
   a) Die IDAAT vermittelte Adhäsion von Thrombozyten an immobilisiertes Thrombospondin-1 wird durch lösliche Integrine α_{IIb}β₃ (5µg/ml bzw. αᵥβ₃ (5 µg/ml) komplett gehemmt. Die angewandte Methodik wurde in der Beschreibung des Beispiels detailliert dargestellt.
   b) Die IDAAT vermittelte Adhäsion von Thrombozyten an immobilisiertes Vitronectin wird teilweise durch lösliche Integrine α_{IIb}β₃ (5*µ*g/ml) bzw. αᵥβ₃ (5 *µ*g/ml) gehemmt. Die angewandte Methodik wurde in der Beschreibung des Beispiels detailliert dargestellt.
**Abbildung 17**: Die IDAAT vermittelte Adhäsion von Thrombozyten an immobilisiertes TSP-1 wird durch den CD36 spezifischen Antikörper Klon 37, bei gleichzeitiger Blockade des Fc-Rezeptors durch IV.3 vollständig gehemmt. Alleinige Fc-Rezeptor-Blockade hat keinen Einfluß. Die angewandte Methodik wurde in der Beschreibung des Beispiels detailliert dargestellt.
**Abbildung 18:** Die IDAAT vermittelte Thrombozytenadhäsion wurde mit Hirudin (20 U/ml) antikoaguliertem Plättchenreichen Plasma durchgeführt. Die angewandte Methodik wurde in der Beschreibung des Beispiels detailliert dargestellt.
**Abbildung 19:** Die IDAAT vermittelte Adhäsion von Thrombozyten an TSP-1 ist abhängig von zweiwertigen Ionen.
   EDTA (5 mM) hemmt diese IDAAT-Wirkung vollständig. 1 mM Ca²⁺ steigert diese IDAAT Wirkung deutlich. Die angewandte Methodik wurde in der Beschreibung des Beispiels detailliert dargestellt.
**Abbildung 20**: Zusatz von löslichem TSP-1 hemmt die Adhäsion von Thrombozyten an Kollagen dosisabhängig, während IDAAT, welches TSP-1 immobilisiert, die Adhäsion von Thrombozyten an Kollagen dosisabhängig steigert. Die angewandte Methodik wurde in der Beschreibung des Beispiels detailliert dargestellt.
**Abbildung 21**: Zusatz von Monozyten (100/µl) zu Thrombozyten (300.000/µl) steigert die Adhäsion von Thrombozyten an TSP-1, während Zusatz von Erythrozyten (20.000/µl) eine hemmende Wirkung hat. Die angewandte Methodik wurde in der Beschreibung des Beispiels detailliert dargestellt.
**Abbildung 22**: Die IDAAT induzierte Thrombozytenadhäsion wurde durch Inhibitoren der PI-3-Kinase Wortmannin (20 nM) und LY294002 (50 *µ*M) gehemmt. Wortmannin und LY294002 wurden dazu für 10 min vor IDAAT-Zusatz vorinkubiert.
**Abbildung 23:** IDAAT vermittelt die Thrombospondin mediierte Thrombozytenaggregation
   Die Thrombozytenaggregation wurde nach Born 1962 durchgeführt. Zu gelfiltrierten Plättchen (200.000/*µ*l) in Hepes-Tyrode Puffer pH7.4 mit 100 *µ*g/ml Fibrinogen wurde in einer Aggregationsküvette TSP-1 (25 *µ*g/ml) pipettiert. Lösliches TSP-1 löste keine Aggregation aus. Zusatz von IDAAT führte zu einer schwachen Aggregationsreaktion. Gleichzeitige Gabe von IDAAT und löslichem TSP-1 führte zu einer starken Aggregatbildung.
**Abbildung 24:** IDAAT vermittelt die Mikropartikelbildung von Thrombozyten Gelfiltrierte Plättchen (50.000/*µ*l) wurden mit dem TSP-1 Peptid RFYVVMWK (40 µM) aktiviert und IDAAT in steigenden Konzentrationen zugesetzt. Nach 30 min Inkubation wurden Plättchen und aus Plättchenentstandenen Mikropartikel mit anti GPIX-PE markiert und die Zahl der entstandenen Mikropartikel im Verhältnis zu 5000 gezählten Plättchen im Durchflußzytometer gemessen.
**Abbildung 25:** IDAAT vermittelt die TSP-1 Bindung an Endothelzellen
   Die Methodik wurde detailliert in der Beschreibung des Beispiels dargestellt. IDAAT verstärkt die TSP-1 Bindung an Endothelzellen.
**Abbildung 26:** IDAAT steigert die Bindung von Thrombozyten an Vitronectin ummantelte Latex-Beads. Die Methodik wurde detailliert in der Beschreibung des Beispiels dargestellt.
**Abbildung 27:**
   a) IDAAT besteht aus polymerem ATIII, dargestellt mit dem "Rotary-Shadowing"-Elektronenmikroskopieverfahren
   b) Handelsübliches ATIII besteht aus monomären globulären Molekülen; elektronenmikroskopische Aufnahme nach Rotationsbedampfung
   c) IDAAT (1 mg/ml) und TSP-1 (200*µ*g/ml) wurden 1 Stunde bei RT miteinander inkubiert. IDAAT und TSP-1 bilden zusammen große Assoziate; elektronenmikroskopische Aufnahme nach Rotationsbedampfung
   d) Handelsübliches ATIII (1 mg/ml) und TSP-1 (200 µg/ml) wurden 1 Stunde bei RT miteinander inkubiert. Handelsübliches AT III und TSP-1 reagierten nicht miteinander; elektronenmikroskopische Aufnahme nach Rotationsbedampfung
**Abbildung 28:** IDAAT bindet direkt an CD4
   Rekombinantes CD4 (1 *µ*g/100 *µ*l/well) wurde an den Boden einer ELISA-Platte (Nunc-Maxisorb) gebunden. Die Platte wurde mit PBS pH 7,4, 0,5 % Tween 20 gründlich gewaschen und freie Plätze auf der Plastikoberfläche mit 3 % BSA für 1 Stunde bei Raumtemperatur geblockt. Die Platte wurde wieder gewaschen und anschließend (DAAT bzw. handelsübliches ATIII in aufsteigenden Konzentrationen von 0-5 µg/ml für 1 Stunde bei RT zugesetzt. Die ATIII-Lösungen wurden entfernt, die Platte gründlich gewaschen und mit einem polyklonalen monospezifischen Antikörper gegen ATIII aus dem Kaninchen DAKO, Hamburg) in einer Verdünnung von 1:15000 in PBS, 1 % NGS (normal goat serum) inkubiert. Die Platte wurde erneut gewaschen und anschließend mit einem affinitätsgereinigten Antikörper aus der Ziege gegen Kaninchen IgG, der mit Peroxidase konjugiert war (BIORAD, München) in einer Verdünnung von 1:3000 inkubiert. Die Platte wurde wieder mehrfach gewaschen und mit Substratlösung (100 µl/well) (20 mg ortho-Phenyldiamin, 5 % H₂O₂ in einem Puffer aus 12,15 ml 0,1 M Zitronensäure und 12,85 ml 0,2 M Na₂HPO₄ plus 25 ml Aqua dest.) versetzt.
   Die Extinktion bei 405 nm gemessen im ELISA-Photometer spiegelt die Menge an gebundenem Antithrombin wieder. Die Reaktion wurde mit 50 µl/well 4 N H₂SO₄ gestoppt und die Extinktion bei 490 nm gemessen. Die Reaktion zeigt deutlich, dass IDAAT nicht nur TSP-1 vermittelt, sondern auch direkt an CD4 binden kann.

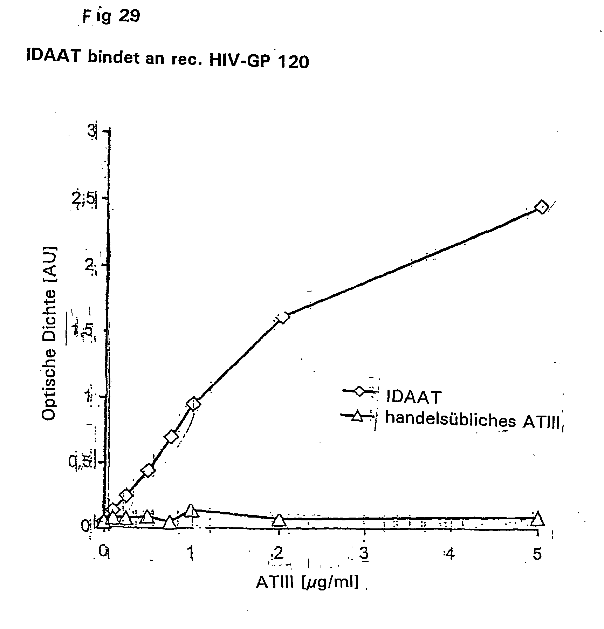

**Abbildung 29:** IDAAT bindet direkt an HIV-GP120
   Rekombinantes HIV-GP120 (1 *µ*g/100 *µ*l/well) wurde an den Boden einer ELISA-Platte (Nunc-Maxisorb) gebunden. Die Platte wurde mit PBS pH 7,4, 0,05 % Tween 20 gründlich gewaschen und freie Plätze auf der Plastikoberfläche mit 3 % BSA für 1 Stunde bei Raumtemperatur geblockt.
   Die Platte wurde wieder gewaschen und anschließend IDAAT bzw. handelsübliches ATIII in aufsteigenden Konzentrationen von 0 - 5*µ*g/ml für 1 Stunde bei RT zugesetzt. Die ATIII-Lösungen wurden entfernt, die Platte gründlich gewaschen und mit einem polyklonalen monospezifischen Antikörper gegen ATIII aus dem Kaninchen (DAKO, Hamburg) in einer Verdünnung von 1 : 15000 in PBS, 1 % NGS (normal goat serum) inkubiert. Die Platte wurde erneut gewaschen und anschließend mit einem affinitätsgereinigten Antikörper aus der Ziege gegen Kaninchen IgG, der mit Peroxidase konjugiert war (BIORAD, München), in einer Verdünnung von 1 : 3000 inkubiert. Die Platte wurde wieder mehrfach gewaschen und mit Substratlösung (100 µl/well) (20 mg ortho-Phenyldiamin, 5 % H₂O₂ in einem Puffer aus 12,15 ml 0.1 M Zitronensäure und 12,85 ml 0.2 M Na₂HPO₄ plus 25 ml Aqua dest.) versetzt.
   Die Extinktion bie 405 nm gemessen im ELISA-Photometer spiegelt die Menge an gebundenem Antithrombin wieder. Die Reaktion wurde mit 50 µl/well 4 N H₂SO₄ gestoppt und die Extinktion bei 490 nm gemessen. Die Reaktion zeigt deutlich, dass IDAAT nicht nur TSP-1 vermittelt, sondern auch direkt an HIV-GP120 binden kann.

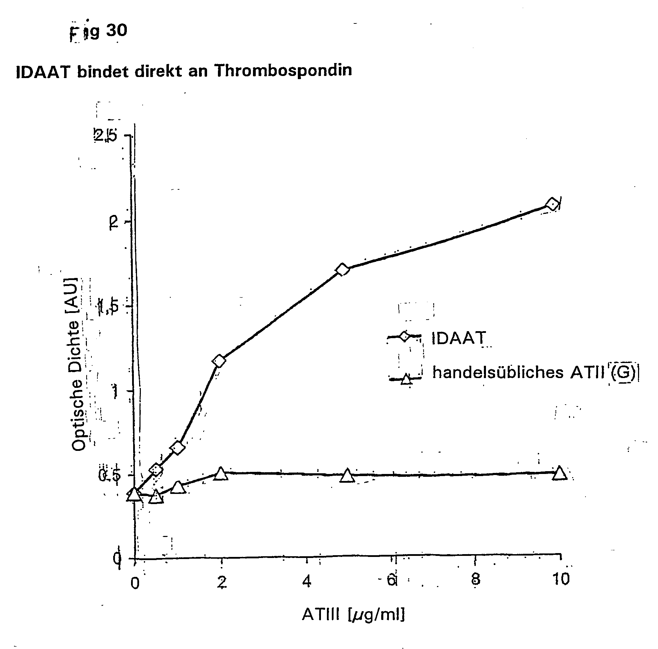

**Abbildung 30:** IDAAT bindet direkt an Thrombospondin
   Gereinigtes Thrombospandin-1 (1 µg/100 µl/well) wurde an den Boden einer ELISA-Platte (Nunc-Maxisorb) gebunden. Die Platte wurde mit PBS pH 7,4, 0,05 % Tween 20 gründlich gewaschen und freie Plätze auf der Plastikoberfläche mit 3 % BSA für 1 Stunde bei Raumtemperatur geblockt. Die Platte wurde wieder gewaschen und anschließend IDAAT bzw. handelsübliches ATIII in aufsteigenden Konzentrationen von 0 - 5 *µ*g/ml für 1 Stunde bei RT zugesetzt. Die ATIII-Lösungen wurden entfernt, die Platte gründlich gewaschen und mit einem polyklonalen monospezifischen Antikörper gegen ATIII aus dem Kaninchen (DAKO, Hamburg) in einer Verdünnung von 1 : 15000 in PBS, 1 % NGS (normal goat serum) inkubiert. Die Platte wurde erneut gewaschen und anschließend mit einem affinitätsgereinigten Antikörper aus der Ziege gegen Kaninchen IgG, der mit Peroxidase konjugiert war (BIORAD, München), in einer Verdünnung von 1 : 3000 inkubiert. Die Platte wurde wieder mehrfach gewaschen und mit Substratlösung (100 µl/well) (20 mg ortho-Phenyldiamin, 5 % H₂O₂ in einem Puffer aus 12,15 ml 0.1 M Zitronensäure und 12,85 ml 0.2 M Na₂HPO₄ plus 25 ml Aqua dest.) versetzt.
   Die Extinktion bei 405 nm gemessen im ELISA-Photometer spiegelt die Menge an gebundenem Antithrombin wieder. Die Reaktion wurde mit 50 *µ*l/well 4 N H₂SO₄ gestoppt und die Extinktion bei 490 nm gemessen. Die Reaktion zeigt deutlich, dass IDAAT direkt an TSP-1 binden kann.

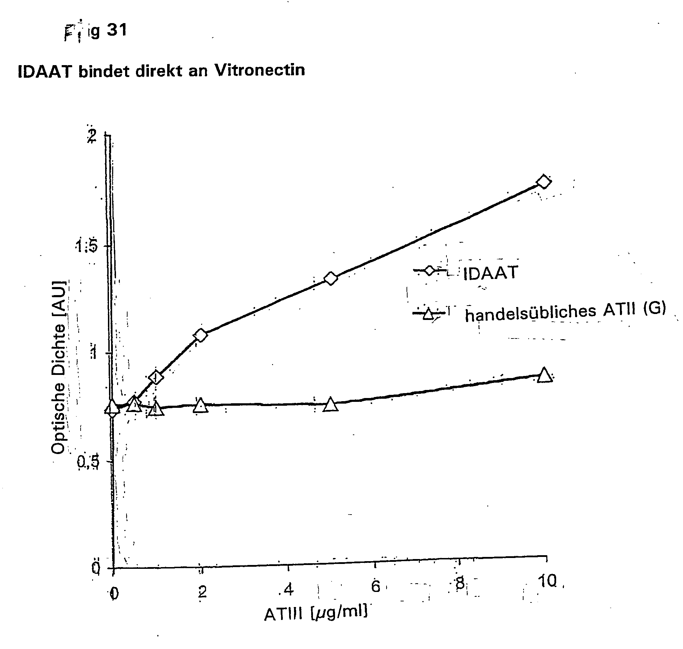

**Abbildung 31:** IDAAT bindet direkt an Vitronectin (aktive Form)
   Vitronectin (1 µg/100 µl/well) wurde an den Boden einer ELISA-Platte (Nunc-Maxisorb) gebunden. Die Platte wurde mit PBS pH 7,4, 0,05 % Tween 20 gründlich gewaschen und freie Plätze auf der Plastikoberfläche mit 3 % BSA für 1 Stunde bei Raumtemperatur geblockt. Die Platte wurde wieder gewaschen und anschließend IDAAT bzw. handelsübliches ATIII in aufsteigenden Konzentrationen von 0 - 5 *µ*g/ml für 1 Stunde bei RT zugesetzt. Die ATIII-Lösungen wurden entfernt, die Platte gründlich gewaschen und mit einem polyklonalen monospezifischen Antikörper gegen ATIII aus dem Kaninchen (DAKO, Hamburg) in einer Verdünnung von 1 : 15000 in PBS, 1 % NGS (normal goat serum) inkubiert. Die Platte wurde erneut gewaschen und anschließend mit einem affinitätsgereinigten Antikörper aus der Ziege gegen Kaninchen IgG, der mit Peroxidase konjugiert war (BIORAD, München), in einer Verdünnung von 1 : 3000 inkubiert. Die Platte uwrde wieder mehrfach gewaschen und mit Substratlösung (100 µl/well) (20 mg ortho-Phenyldiamin, 5 % H₂O₂ in einem Puffer aus 12,15 ml 0,1 M Zitronensäure und 12,85 ml 0.2 M Na₂HPO₄ plus 25 ml Aqua dest.) versetzt. Die Extinktion bei 405 nm gemessen im ELISA-Photometer spiegelt die Menge an gebundenem Antithrombin wieder. Die Reaktion wurde mit 50 µl/well 4 N H₂SO₄ gestoppt und die Extinktion bei 490 nm gemessen. Die Reaktion zeigt deutlich, dass IDAAT direkt an aktives Vitronectin binden kann.
   **Herstellung von IDAAT:**

### Beispiel 1

Im beschriebenen Sinne nicht funktionsfähiges, nicht aktiviertes Antithrombin III wurde entweder von Calbiochem, Sigma, Enzyme Research Laboratories, Pharmacia & Upjohn, Aventis, Baxter oder Grifols bezogen oder aus humanem Plasma gereinigt. Die Antithrombin-Präparate wurden gegen Phosphat-gepufferte Saline (PBS) pH 7,4 umgepuffert. 348 µg reines Antithrombin wurde mit PBS pH 7,4 und 0,1 mM EDTA auf ein Volumen von 1 ml gebracht und die Lösung auf Eis gekühlt. Kaltes NaOCl (832 µg) wurde in einem Volumen von 10 µl zugesetzt und der Ansatz für 10 Minuten auf Eis inkubiert. Die Reaktion wurde durch sofortige Gelfiltration bei 4 °C über Sephadex G25 (PD10-Säulen) terminiert.

### Beispiel 2

Handelsübliches, nicht aktiviertes Antithrombin lll wurde gegen PBS pH 8,0 umgepuffert. 500 µg reines Antithrombin lll wurde mit 50 µg Neutrophile Granulozyten-Elastase (HNE) (human, gelöst in 50 µl Puffer) versetzt und der Ansatz (500 *µ*l Volumen) für 16 Stunden bei 37 °C inkubiert. Die Reaktion wurde mt 1 mM (Endkonzentration) Phenylmethylsulfonylfluorid (PMSF) gestoppt und das Antithrombin III mit dem Centricon-Verfahren gegen PBS/0,1 mM EDTA pH 7,4 umgepuffert. Anschließend erfolgte eine Oxidation mit NaOCl wie im Beispiel 1 beschrieben.

### Beispiel 3

Handelsübliches, nicht aktiviertes Antithrombin III wurde gegen PBS pH 8,0 umgepuffert. 500 µg reines Antithrombin III wurde mit 12,4 µg Matrixmetalloproteinase-2 (MMP-2) (gelöst in 0,9 % NaCl) in 25 mM Tris/HCl/30 mM NaCl/10 mM Ca²⁺-Puffer angesetzt. Um die MMP-2 zu aktivieren, wurde sie für 2 Stunden bei RT mit 1 mM APMA (4-aminophenylmercuric acetate) vorbehandelt. Der Ansatz (500 *µ*l Volumen) wurde für 16 Stunden bei 37°C inkubiert. Das Antithrombin III wurde mit dem Centricon-Verfahren gegen PBS pH 7,4 umgepuffert.

### Beispiel 4

Handelsübliches, nicht aktiviertes Antithrombin III wurde gegen PBS pH 8,0 umgepuffert. 200 µg reines Antithrombin III wurde mit Defensin 2 (HNP-2, gelöst in 0,9 % NaCl, Endkonzentration 10 µM) in PBS pH 8,0 für 1 Stunde bei RT inkubiert.

### Referenzen

1. Armant M, Avice MN, Hermann P, Rubio M, Kiniwa M, Delespesse G, Sarfati M: CD47 ligation selectively downregulates human interleukin 12 production. J.Exp.Med. 190:1175-1182,1999
2. Asch AS, Liu 1, Briccetti FM, Barnwell JW, Kwakye-Berko F, Dokun A, Goldberger J, Pernambuco M: Analysis of CD36 binding domains: ligand specificity controlled by dephosphorylation of an ectodomain. Science 262: 1436-1440, 1993
3. Barnes DA, Tse J, Kaufhold M, Owen M, Hesseigesser J, Strieter R, Horuk R, Perez HD: Polyclonal antibody directed against human RANTES ameliorates disease in the Lewis rat adjuvant-induced arthritis model. J.Clin.Invest 101: 2910-29199 1998
4. Born GV: [The blood platelets in thrombogenesis. The mechanism and inhibition of the aggregation of blood platelets]. Actual.Pharmacol.(Paris) 18: 17-32, 1965
5. Bornstein P, Devarayalu S, Li P, Disteche CM, Framson P: A second thrombospondin gene in the mouse is similar in organization to thrombospondin 1 but does not respond to serum. Proc.Natl.Acad.Sci.U.S.A 88: 8636-8640,1991
6. Bornstein P: Thrombospondins: structure and regulation of expression [published erratum appears in FASEB J 1993 Jan; 7(1):237]. FASEB J. 6: 3290-3299, 1992
7. Chung J, Gao AG, Frazier WA: Thrombspondin acts via integrinassociated protein to activate the platelet integrin alphallbbeta 3. J.Biol.Chem. 272: 14740-14746, 1997
8. Chung J, Wang XQ, Lindberg FP, Frazier WA: Thrombospondin-1 acts via IAP/CD47 to synergize with collagen in alpha2betal-mediated platelet activation. Blood 94: 642-6489 1999
9. Crawford SE, Stellmach V, Murphy-Ullrich JE, Ribeiro SM, Lawler J, Hynes RO, Boivin GP, Bouck N: Thrombospondin-1 is a major activator of TGF-betal in vivo. Cell 93: 1159-1170, 1998
10. Crombie R, Sitverstein RL, MacLow C, Pearce SFA, Nachman RL, Laurence J: Identification of a CD36-related thrombospondin 1-binding domain in HIV- 1 envelope glycoprotein gpl20: relationship to HIV-1-specific inhibitory factors in human saliva. J.Exp.Med. 187: 25-35, 1998
11. Dardik R, Lahav J: Functional changes in the conformation of thrombospondin-1- during complexation with fibronectin or heparin. Exp.Cell Res. 248: 407-414,1999
12. Demeure CE, Tanaka H, Mateo V, Rubio M, Delespesse G, Sarfati M: CD47 engagement inhibits cytokine production and maturation of human dendritic cells. J.Immunol. 164: 2193-2199, 2000
13. Dickneite G, Paques EP: Reduction of mortality with antithrombin 111 in septicemic rats: a study of Klebsiella pneumoniae induced sepsis. Thromb.Haemost. 69: 98-102, 1993
14. Dörmann D, Kardoeus J, Zimmermann RE, Kehrel B: Flow cytometric analysis of agonist-Induced annexin V, factor Va and factor Xa binding to human platelets. Platelets 9: 171-1771 1998
15. Fadok VA, Bratton DL, Konowal A, Freed PW, Westcott JY, Henson PM: Macrophages that have ingested apoptotic cells in vitro inhibit proinflammatory cytokine production through autocrine/paracrine mechanisms involving TGF-beta, PGE2, and PAF. J.Clin.Invest 101: 890-8982, 1998
16. Galvin NJ, Dixit VM, O'Rourke KM, Santoro SA, Grant GA, Frazier WA: Mapping of epitopes for monoclonal antibodies against human platelet thrombospondin with electron microscopy and high sensitivity amino acid sequencing. J.Cell Biol. 101: 1434-1441, 1985
17. Geiser AG, Letterio JJ, Kulkarni AB, Karlsson S, Roberts AB, Sporn MB: Transforming growth factor beta 1 (TGF.beta 1 controls expression o major histocompatibility genes in the postnatal mouse: aberrant histocompatibility antigen expression in the pathogenesis of the TGF beta 1 null mouse phenotype. Proc.Natl.Acad.Sci.U.S.A 90: 9944-9948, 1993
18. Higazi AA, Upson RH, Cohen RL, Manuppello J, Bognacki J, Henkin J, McCrae KR, Kounnas MZ, Strickland DK, Preissner KT, Lawier J, Cines DB: Interaction of single-chain urokinase with its receptor induces the appearance and disappearance of binding epitopes within the resultant complex for other cell surface proteins. Blood 88: 542-5511, 1996
19. Iruela-Arispe ML, Lombardo M, Krutzsch HC, Lawler J, Roberts DD: Inhibition of angiogenesis by thrombospondin-1 is mediated by 2 independent regions within the type 1 repeats. Circulation 100: 1423-14311, 1999
20. Jander R, Troyer D, Rauterberg J: A collagen-like glycoprotein of the extracellular matrix is the undegraded form of type VI collagen. Biochemistry 23: 3675-3681, 1984
21. Jimenez B, Volpert OV, Crawford SE, Febbraio M, Silverstein RL, Bouck N: Signals leading to apoptosis-dependent inhibition of neovascularization by thrombospondin-1. Nat.Med. 6: 41-48, 2000
22. Kainoh M, Imai R, Umetsu T, Hattori M, Nishio S: Prostacyclin and beraprost sodium as suppressors of activated rat polymorphonuclear leukocytes. Biochem.Pharmacol. 39: 477-4841, 1990
23. Kaplan HJ, Leibole MA, Tezei T, Ferguson TA: Fas ligand (CD95 ligand) controls angiogenesis beneath the retina. Nat.Med. 5: 292-297, 1999
24. Kappe S, Bruderer T, Gantt S, Fujioka H, Nussenzweig V, Menard R: Conservation of a gliding motility and cell invasion machinery in Apicomplexan parasites. J.Cell Biol. 147: 937-9441, 1999
25. Kehrel B, Balleisen L, Kokott R, Mesters R, Stenzinger W, Clemetson KJ, van de LJ: Deficiency of intact thrombospondin and membrane glycoprotein la in platelets with defective collagen-induced aggregation and spontaneous loss of disorder. Blood 71: 1074-10787, 1988
26. Kehrel B, Kronenberg A, Schwippert B, Niesing-Bresch D, Niehues U, Tschope D, van de LJ, Clemetson KJ: Thrombospondin binds normally to glycoprotein lllb deficient platelets. Biochem. Biophys. Res. Commun. 179: 985-991, 1991
27. Kehrel B, Flicker E: Thrombospondin in Pathophysiology - Thrombospondin in Relation with Disease Processes, in Lahav J (ed): Thrombospondin. Boca Raton, CRC Press, 1993, pp 199-207
28. Kehrel B, Flicker E, Wigbels B, Osterfeld M, van de LJ, Luscher EF: Thrombospondin measured in whole blood - an indicator of platelet activation. Blood Coagul.Fibrinolysis 7: 202-205, 1996
29. Kehrel B, Wierwille S, Clemetson KJ, Anders O, Steiner M, Knight CG, Farndale RW, Okuma M, Barnes MJ: Glycoprotein VI is a major Collagen receptor for platelet activation: it recognizes the platelet-activating quaternary structure of collagen, whereas CD36, glycoprotein IIb/IIIa, and von Willebrand factor do not. Blood 91: 491-4991, 1998
30. Kielbassa K, Schmitz C, Gerke V: Disruption of endothelial microfilaments selectively reduces the transendothelial migration of monocytes. Exp.Cell Res. 243: 129-141, 1998
31. Kronenberg A, Grahl H, Kehrel B: Human platelet CD36 (BPIIIb, GPIV) binds to cholesteryl-hemisuccinate and can be purified by a simple two-step method making use of this property. Thromb. Haemost. 79: 1021-1024, 1998
32. Kulkarni AB, Huh CG, Becker D, Geiser A, Lyght M, Flanders KC, Roberts AB, Sporn MB, Ward JM, Karlsson S: Transforming growth factor beta 1 null mutation in mice causes excessive inflammatory response and early death. Proc.NatI.Acad.Sci.U.S.A 90: 770-774, 1993
33. Kulkarni AB, Karisson S: Inflammation and TGF beta 1: lessons from the TGF beta 1 null mouse. Res.Immunol. 148: 453-456, 1997.
34. Lawler J, Hynes RO: The structure of human thrombospondin, an adhesive glycoprotein with multiple calcium-binding sites and homologies with several different proteins. J.Cell Biol. 103: 1635-1648, 1986
35. Lawler J: The structural and functional properties of thrombospondin. Blood 67: 1197-1209, 1986
36. Lawler J, Weinstein R, Hynes RO: Cell attachment to thrombospondin: the role of ARG-GLY-ASP, calcium, and integrin receptors. J.Cell Biol. 107: 2351-2361, 1988
37. Lawler J, Duquette M, Urry L, McHenry K, Smith TF: The evolution of the thrombospondin gene family. J.Mol.Evol. 36: 509-516, 1993
38. Lawler J, Sunday M, Thibert V, Duquette M, George EL, Rayburn H, Hynes RO: Thrombospondin-1 is required for normal murine pulmonary homeostasis and its absence causes pneumonia. J.Clin.Invest 101: 982-9921, 1998
39. Letterio JJ, Geiser AG, Kulkarni AB, Dang H, Kong L, Nakabayashi T, Mackall CL, Gress RE, Roberts AB: Autoimmunity associated with TGF-betal-deficiency in mice is dependent on MHC class 11 antigen expression. J.Clin.Invest 98: 2109-2119, 1996
40. Leung LL, Li WX, McGregor JL, Albrecht G, Howard RJ: CD36 peptides enhance or inhibit CD36-thrombospondin binding. A two-step process of ligand-receptor interaction. J.Biol.Chem. 267: 18244-18250, 1992
41. Li DQ, Lundberg F, Ljungh A: Binding of von Willebrand factor by coagulase-negative staphylococci. J.Med.Microbiol. 49: 217-2259, 2000
42. Lindstedt KA, Kokkonen JO, Kovanen PT: Soluble heparin proteoglycans released from stimulated mast cells induce uptake of low density lipoproteins by macrophages via scavenger receptor-mediated phagocytosis. J.Lipid Res. 33: 65-75, 1992
43. Mateo V, Lagneaux L, Bron D, Biron G, Armant M, Delespesse G, Sarfati M: CD47 ligation induces caspase-independent cell death in chronic lymphocytic leukemia. Nat. Med. 5: 1277-1284, 1999
44. Munjai ID, Crawford DR, Blake DA, Sabet MD, Gordon SR: Thrombospondin: biosynthesis, distribution, and changes associated with wound repair in corneal endothelium [published erratum appears in Eur J Cell Biol 1991 Aug. 55(2):IV]. Eur.J.Cell Bloi. 52: 252-263, 1990
45. Murphy-Ullrich JE, Gurusiddappa S, Frazier WA, Hook M: Heparinbinding peptides from thrombospondins 1 and 2 contain focal adhesionlabilizing activity. J.Biol.Chem. 268: 26784-26789, 1993
46. Nakamura T, Amano A, Nakagawa 1, Hamada S: Specific interactions between Porphyromonas gingivalis fimbriae and human extracellular matrix proteins. FEMS Microbiol.Lett. 175: 267-272, 1999
47. Patthy L: Detecting distant homologies of mosaic proteins. Analysis of the sequences of thrombomodulin, thrombospondin complement components C9, C8 alpha and C8 beta, vitronectin and plasma cell Membrane glycoprotein PC-1. J.Mol.Biol. 202: 689-6969, 1988
48. Roberts DD, Sherwood JA, Spitalnik SL, Panton LJ, Howard RJ, Dixit VM, Frazier WA, Miller LH, Ginsburg V: Thrombospondin binds falciparum malaria parasitized erythrocytes and may mediate cytoadherence. Nature 318: 64-66, 1985
49. Roberts DD: Regulation of tumor growth and metastasis by thrombospondin-1. FASEB J. 10: 1183-1191, 1996
50. Santoro SA, Zutter MM, Wu JE, Staatz WD, Saelman EU, Keely PJ: Analysis of collagen receptors. Methods Enzymol. 245: 147-1839, 1994
51. Savill J, Hogg N, Ren Y, Haslett C: Thrombospondin cooperates with CD36 and the vitronectin receptor in macrophage recognition of neutrophils undergoing apoptosis. J.Clin.Invest 90: 1513-1522, 1992
52. Schultz-Cherry S, Murphy-Ullrich JE: Thrombospondin causes activation of latent transforming growth factor- beta secreted by endothelial cells by a novel mechanism [published erratum appears in J Cell Biol 1993 Sep; 122(5):following 1143]. J.Cell Biol. 122: 923-932, 1993
53. Schultz-Cherry S, Lawier J, Murphy-Ullrich JE: The type 1 repeats of thrombospondin 1 activate latent transforming growth factor-beta. J.Biol.Chem. 269: 26783-26788, 1994
54. Shafiee A, Penn JS, Krutzsch HC, Inman JK, Roberts DD, Blake DA: Inhibition of retinal angiogenesis by peptides derived from thrombospondin-1. Invest Ophthalmol.Vis.Sci. 41: 2378-23881, 2000
55. Shull MM, Ormsby 1, Kier AB, Pawlowski S, Diebold RJ, Yin M, Allen R, Sidman C, Proetzei G, Calvin D: Targeted disruption of the mouse transforming growth factor-beta 1 gene results in multifocal inflammatory disease. Nature 359: 693-699, 1992
56. Silverstein RL, Nachman RL: Thrombospondin-plasminogen interactions: modulation of plasmin Generation. Semin.Thromb.Hemost. 13: 335-342, 1987
57. Silverstein RL, Nachman RL, Pannell R, Gurewich V, Harpel PC: Thrombospondin forms complexes with single-chain and two-chain forms of urokinase [published erratum appears in J Biol Chem 1990 Sep 15; 265(26): 16025). J.Biol.Chem. 265: 11289-11294, 1990
58. Sozzani S, Molino M, Locati M, Luini W, Cerletti C, Vecchi A, Mantovani A: Receptor-activated calcium influx in human monocytes exposed to monocyte chemotactic protein-1 and related cytokines. J.Immunol. 150: 1544-15531 1993
59. Stangl K, Dschietzig T, Alexiou K, Brunner F: Antithrombin increases pulmonary endothelins: inhibition by heparin and Ca2 + channel antagonism. Eur.J.Pharmacol. 370: 57-61, 1999
60. Steinhauser ML, Hogaboam CM, Lukacs NW, Strieter RM, Kunkel SL: Multiple roles for IL-12 in a model of acute septic peritonitis. J.Immunol. 162: 5437-5443, 1999
61. Stern M, Savill J, Hasiett C: Human monocyte-derived macrophage phagocytosis of senescent eosinophils undergoing apoptosis. Mediation by alpha v beta 3/CD36/thrombospondin recognition mechanism and lack of phlogistic response. Am.J.Pathol. 149: 911-921, 1996
62. Sulaiman IM, Lal AA, Arrowood MJ, Xiao L: Bialielic polymorphism in the intron region of beta-tubulin gene of Cryptosporidium parasites. J. Parasitol. 85: 154-157, 1999
63. Uchiba M, Okajima K, Murakami K, Okabe H, Takatsuki K: Attenuation of endotoxin-induced pulmonary vascular injury by antithrombin 111. Am.J.Physiol 270: L921-L930,1996
64. Watkins SC, Lynch GW, Kane LP, Slayter HS: Thrombospondin expression in traumatized skeletal muscle. Correlation of appearance with post-trauma regeneration. Cell Tissue Res. 261: 73-84t i990
65. Wengelnik K, Spaccapelo R, Naitza S, Robson KJ, Janse CJ, Bistoni F, Waters AP, Crisanti A: The A-domain and the thrombospondin-related motif of Plasmodium falciparum TRAP are implicated in the invasion process of mosquito sallvary glands. EMBO J. 18: 5195-5204, 1999
66. Yamauchi T, Umeda F, Inoguchi T, Nawata H: Antithrombin III stimulates prostacyclin production by cultured aortic endothelial cells. Biochem.Biophys.Res.Commun. 163: 1404-1411, 1989
67. Yatohgo T, Izumi M, Kashiwagi H, Hayashi M: Novel purification of vvitronectin from human plasma by heparin affinity chromatography. Cell Struct. Funct. 13: 281-292, 1988
68. Yehualaeshet T, O'Connor R, Green-Johnson J, Mai S, Silverstein R, Murphy-Ullrich JE, Khalii N: Activation of rat alveolar macrophage-derived latent transforming growth factor beta-1 by plasmin requires interaction with thrombospondin-1 and its cell surface receptor, CD36. Am.J.Pathol. 155: 841-851, 1999

## Patentansprüche

1. Arzneimittel, enthaltend durch Oxidation aktiviertes Antithrombin III (IDAAT) oder IDAAT-Peptide.

2. Arzneimittel nach Anspruch 1,
**dadurch gekennzeichnet,**
**dass** die Oxidation durch Behandlung mit NaOCl erfolgt.

3. Arzneimittel nach Anspruch 1 oder 2,
**dadurch gekennzeichnet,**
**dass** es rekombinantes IDAAT oder Peptide oder Analoga davon enthält.

4. Arzneimittel nach einem der vorhergehenden Ansprüche,
**dadurch gekennzeichnet,**
**dass** es weitere pharmazeutisch akzeptable Hilfs- oder/und Trägersubstanzen enthält.

5. Arzneimittel nach einem der vorhergehenden Ansprüche,
**dadurch gekennzeichnet,**
**dass** es zur lokalen, intradermalen, oberflächlichen, intraperitonalen, intravenösen, oralen oder intramuskulären Verabreichung formuliert ist, oder über Vesikel verabreicht wird.

6. Arzneimittel nach einem der vorhergehenden Ansprüche,
**dadurch gekennzeichnet,**
**dass** es weitere Substanzen, wie z.B. Antibiotika, oder Interaktionspartner des IDAAT im Körper enthält.

7. Arzneimittel nach einem der Ansprüche 1 bis 6 zur Prophylaxe oder Therapie von akuten Infektionen, Sepsis, akuten, chronischen und/oder allergischen Entzündungsreaktionen, für Leukämie, zur Verbesserung der Wundheilung, zur Behandlung von Läsionen im Nervensystem, zur Verbesserung der Blutstillung sowie zur Verhinderung von entzündlich bedingten Gewebeschädigungen, sowie zur allgemeinen Steigerung der Immunabwehr und Inhibition von Entzündungsreaktionen.

8. Arzneimittel nach Anspruch 7,
**dadurch gekennzeichnet,**
**dass** als akute Infektion eine HIV-Infektion behandelt wird.

9. Arzneimittel nach Anspruch 7,
**dadurch gekennzeichnet,**
**dass** insbesondere chronisch lymphatische Leukämie behandelt wird.

10. Arzneimittel nach Anspruch 7,
**dadurch gekennzeichnet,**
**dass** es ebi Patienten mit hohem Infektionsrisiko, wie nach Operationen mit großer Infektionsgefahr, Polytramen, Verbrennungen, Intoxikationen unter Chemotherapie, bei immunsupprimierten Patienten und Patienten mit prädisponierter Abwehrschwäche eingesetzt wird.

11. Arzneimittel nach Anspruch 7,
**dadurch gekennzeichnet,**
**dass** es zur Blutstillung, insbesondere bei Patienten mit angeborenen oder erworbenen Blutgerinnungsstörunegn oder angeborenen oder erworbenen Thrombozytopathien, unter Antikoagulationstherapie oder Thrombozytenprophylaxe oder bei Operationen unter Herzlungenmaschine eingseetzt wird.

12. Arzneimittel nach Anspruch 7,
**dadurch gekennzeichnet,**
**dass** es bei Reperfusionen, Organtransplantationen oder allergischen Reaktionen eingesetzt wird.

13. Arzneimittel nach Anspruch 7,
**dadurch gekennzeichnet,**
**dass** parasitäre Erkrankungen, insbesondere Malaria tropica, behandelt werden.

## Claims

1. Pharmaceutical preparation containing antithrombin III (IDAAT) or IDAAT-peptides activated by oxidation.

2. Pharmaceutical preparation according to claim 1,
**characterized in that**
the oxidation is carried out by treatment with NaOCl.

3. Pharmaceutical preparation according to claim 1 or 2,
**characterized in that**
it contains recombinant IDAAT or peptides or analogues thereof.

4. Pharmaceutical preparation according to one of the previous claims,
**characterized in that**
it contains additional pharmaceutically acceptable auxiliary substances or/and excipients.

5. Pharmaceutical preparation according to one of the previous claims,
**characterized in that**
it is formulated for local, intradermal, superficial, intraperitoneal, intravenous, oral or intramuscular administration or it is administered by means of vesicles.

6. Pharmaceutical preparation according to one of the previous claims,
**characterized in that**
it contains additional substances such as antibiotics or interaction partners of IDAAT in the body.

7. Pharmaceutical preparation according to one of the claims 1 to 6 for the prophylaxis or treatment of acute infections, sepsis, acute, chronic and/or allergic inflammatory reactions, for leukaemia, to improve wound healing, to treat lesions in the nervous system, to improve blood haemostasis and prevent tissue damage caused by inflammation and to generally increase immune defence and to inhibit inflammatory reactions.

8. Pharmaceutical preparation according to claim 7,
**characterized in that**
a HIV infection is treated as the acute infection.

9. Pharmaceutical preparation according to claim 7,
**characterized in that**
in particular chronic lymphatic leukaemia is treated.

10. Pharmaceutical preparation according to claim 7,
**characterized in that**
it is used in patients with a high risk of infection such as after operations with a high risk of infection, polytrauma, burns, intoxication, patients under chemotherapy, in immunosuppressed patients and patients with a predisposition for immune deficiency.

11. Pharmaceutical preparation according to claim 7,
**characterized in that**
it is used for blood haemostasis in particular in patients with congenital or acquired disorders of blood coagulation or congenital or acquired thrombocytopathies, in patients undergoing anticoagulation therapy or thrombocyte prophylaxis or in operations using a heart-lung machine.

12. Pharmaceutical preparation according to claim 7,
**characterized in that**
it is used in reperfusions, organ transplantations or allergic reactions.

13. Pharmaceutical preparation according to claim 7,
**characterized in that**
parasitic diseases and in particular Malaria tropica are treated.

## Revendications

1. Médicament contenant de l'antithrombine III activée par oxydation (IDAAT) ou un peptide de l'IDAAT.

2. Médicament selon la revendication 1, **caractérisé en ce que** l'oxydation s'effectue par traitement avec du NaOCl.

3. Médicament selon la revendication 1 ou 2, **caractérisé en ce qu'**il contient l'IDAAT recombinant ou un peptide ou un analogue de celle-ci.

4. Médicament selon l'une quelconque des revendications précédentes, **caractérisé en ce qu'**il contient d'autres adjuvants et/ou véhicules pharmaceutiquement acceptables.

5. Médicament selon l'une quelconque des revendications précédentes, **caractérisé en ce qu'**il est formulé pour l'administration locale, intradermique, superficielle, intrapéritonéale, intraveineuse, orale ou intramusculaire ou est administré par le biais de vésicules.

6. Médicament selon l'une quelconque des revendications précédentes, **caractérisé en ce qu'**il contient d'autres substances comme par exemple des antibiotiques ou un partenaire d'interaction de l'IDAAT dans l'organisme.

7. Médicament selon l'une quelconque des revendications 1 à 6, pour la prophylaxie ou le traitement d'infections aiguës, de septicémie, de réactions inflammatoires chroniques et/ou allergiques aiguës, pour la leucémie, pour améliorer la cicatrisation des plaies, pour le traitement des lésions du système nerveux, pour améliorer l'hémostase et pour empêcher les dommages tissulaires de nature inflammatoire et pour augmenter de manière générale les défenses immunitaires et inhiber des réactions inflammatoires.

8. Médicament selon la revendication 7, **caractérisé en ce que** l'on traite comme infection aiguë, une infection au VIH.

9. Médicament selon la revendication 7, **caractérisé en ce que** l'on traite en particulier la leucémie lymphatique chronique.

10. Médicament selon la revendication 7, **caractérisé en ce qu'**il est utilisé chez les patients présentant un risque d'infection élevé tel qu'après des opérations présentant un grand risque d'infection, des polytraumatismes, des brûlures, des intoxications sous chimiothérapie, chez les patients immunodéprimés et les patients prédisposés à une défaillance immunitaire.

11. Médicament selon la revendication 7, **caractérisé en ce qu'**il est utilisé pour l'hémostase, en particulier chez les patients présentant des troubles innés ou acquis de la coagulation ou des thrombopathies innées ou acquises, les patients sous traitement anticoagulant ou en prophylaxie thrombocytaire ou en cas d'opérations avec circulation extracorporelle.

12. Médicament selon la revendication 7, **caractérisé en ce qu'**il est utilisé dans les cas de reperfusion, de greffes d'organes ou de réactions allergiques.

13. Médicament selon la revendication 7, **caractérisé en ce que** l'on traite des maladies parasitaires, en particulier le paludisme.
